# EUROPEAN PATENT SPECIFICATION

(11) **EP 3 970 712 B1**
(45) Date of publication and mention of the grant of the patent: **18.11.2026**
(21) Application number: 21203394.8
(22) Date of filing: 05.06.2017
(51) Int. Cl.: A61K 31/135, A61K 31/137, A61K 31/343, A61K 31/41, A61K 31/423, A61K 31/4525, A61K 31/195, A61K 31/4045, A61K 31/417, A61K 31/6615, A61K 31/381, A61P 25/24

(54) **SOLID ORAL DOSAGE FORMS OF 2R,6R-HYDROXYNORKETAMINE OR PRODRUGS THEREOF IN COMBINATION WITH A SEROTONIN MODULATOR FOR USE IN THE TREATMENT OF A DEPRESSIVE DISORDER**
FESTE ORALE DARREICHUNGSFORMEN VON 2R,6R-HYDROXYNORKETAMIN ODER PRODRUGS DAVON IN KOMBINATION MIT EINEM SEROTONINMODULATOR ZUR VERWENDUNG BEI DER BEHANDLUNG VON DEPRESSIONEN
FORMES POSOLOGIQUES SOLIDES ORALES DE 2R,6R-HYDROXYNORKÉTAMINE OU LEURS PRODROGUES EN COMBINAISON AVEC UN MODULATEUR DE SÉROTONINE POUR UTILISATION DANS LE TRAITEMENT D'UN TROUBLE DÉPRESSIF

(30) Priority: 03.06.2016 GB 201609790; 05.12.2016 GB 201620690
(43) Date of publication of application: 23.03.2022
(62) Document number(s) of the earlier application(s) in accordance with Art. 76 EPC: 17728640.8 / 3 463 323
(73) Proprietor: Cybin UK Ltd, London WC2N 4JF (GB)
(72) Inventor: RANDS, Peter, Greater London (GB); LAYZELL, Marie, Greater London (GB); JOEL, Zelah, Greater London (GB); ARMSTRONG, Alan, Greater London (GB); MYERSON, Richard, Chapel-en-le-Frith (GB)
(74) Representative: Patent Boutique LLP

(56) References cited:
- WO-A1-2013/056229
- PANOS ZANOS ET AL: "NMDAR inhibition-independent antidepressant actions of ketamine metabolites.(includes Methods)", NATURE,, vol. 533, 26 May 2016 (2016-05-26), pages 481, XP002773197, DOI: 10.1038/NATURE17998

## Description

### FIELD OF THE INVENTION

This invention relates to solid oral dosage forms of metabolites of ketamine and prodrugs of metabolites of ketamine and analogues thereof, including any pharmaceutically acceptable salt of the foregoing, wherein the solid dosage form is for administration in combination with a serotonin modulator, and the combination is for use as a first-line therapy in the treatment of a depressive disorder in a patient. In particular the invention relates to solid oral dosage forms comprising the ketamine metabolite 2R,6R-hydroxynorketamine or prodrugs thereof for use as a first-line therapy in the treatment of a depressive disorder in a patient.

### BACKGROUND TO THE INVENTION

Depressive disorders are a category of mood disorders that cause a persistent feeling of sadness and loss of interest in the sufferer. Symptoms of depressive disorders include feelings of helplessness and hopelessness, loss of interest in daily activities, appetite or weight changes, sleep changes, anger or irritability, loss of energy, self-loathing, reckless behaviour, concentration problems, and unexplained aches and pains.

Depressive disorders are generally treated with psychotherapy, medication, or both.

Psychotherapy can help people with depressive disorders. Effective psychotherapies include Cognitive Behavioural Therapy (CBT), Self-Help or Support Groups, and Stress-Management Techniques.

CBT is a type of psychotherapy that can help patients with depressive disorders. It teaches a patient different ways of thinking, behaving, and helps the patient exercise control on their mood. CBT can also help patients learn and practice social skills which can assist with depressive disorders.

CBT may be conducted individually or with a group of patients who have similar problems. Group therapy is particularly effective for social depressive disorder. Often "homework" is assigned for participants to complete between sessions.

Meditation can help patients with depressive disorders calm themselves and may enhance the effects of therapy.

In some cases, medications are used as the initial treatment of a depressive disorder; more commonly medications are used if there is insufficient response to a course of psychotherapy.

The most common classes of medications used to combat depressive disorders, also referred to as antidepressant agents, are serotonin modulators.

Serotonin modulators can be effective in treating depressive disorders, but they take several weeks to start working and may cause side effects such as headache, nausea, or difficulty sleeping. In order to minimize the impact of serotonin modulator side effects, typical dosing regimens start a patient on a low dose of the medication and increase the dose slowly over time.

In May 2016, Panos Zanos and colleagues (Nature, 533, 481-486) demonstrated preclinical antidepressant efficacy of 2R,6R-hydroxynorketamine. This follows earlier demonstration of biological activity of 2R,6R-hydroxynorketamine published in WO2013/056229A1.

There is a need in the art to find new and improved therapies for the treatment of depressive disorders. The present invention provides novel therapies for the treatment of depressive disorders. These therapies have the potential to provide greater efficacy than current treatment regimens, achieve greater compliance and/or improve outcomes in patients without introducing unacceptable side-effect liability.

### SUMMARY OF THE INVENTION

A first aspect of the present invention provides a solid oral dosage form comprising a compound selected from 2R,6R-hydroxynorketamine or a pharmaceutically acceptable salt thereof or a prodrug of 2R,6R-hydroxynorketamine having a structure of Formula Ib (as defined in claim 1) or a pharmaceutically acceptable salt thereof, wherein the solid oral dosage form is for administration in combination with a serotonin modulator, for use as a first-line therapy in the treatment of a depressive disorder in a patient.

Intravenous infusions of 0.5mg/kg of ketamine hydrochloride administered over a period of 40 mins are known to exhibit potent and rapid anti-depressant effects in treatment-resistant depression patients, reported, for example, in Murrough et al; Anti-depressant Efficacy of Ketamine in Treatment-Resistant Major Depression: A Two-Site Randomized Controlled Trial; Am J Psychiatry. 1 October 2013; 170(10): 1134-1142.

The use of ketamine hydrochloride in the treatment of mental health is limited by the dosage form in which it is typically provided. Ketamine hydrochloride is approved in a form administrable only by intravenous infusion. This renders it inconvenient and/or inappropriate for use in depressed patients: it is preferable to be able to administer therapy in an out-patient setting.

In preferred embodiments of the first aspect of the present invention, the metabolite of ketamine is a mono-hydroxylated metabolite.

In preferred embodiments of the first aspect of the present invention, the metabolite of ketamine is in the form of an acid-addition salt.

In preferred embodiments of the first aspect of the present invention, the solid oral dosage form comprises 2R,6R-hydroxynorketamine in the form of an acid-addition salt.

In the first aspect of the present invention, the pharmaceutically acceptable salt of 2R,6R-hydroxynorketamine is preferably not 2R,6R-hydroxynorketamine hydrochloride.

In preferred embodiments the first aspect of the present invention the solid oral dosage form comprises the active ingredient 2R,6R-hydroxynorketamine, or a prodrug thereof, or pharmaceutically acceptable salts thereof, wherein the active ingredient is present in an amount equivalent to between 10mg and 100mg of 2R,6R-hydroxynorketamine freebase.

In preferred embodiments of the first aspect of the invention, the metabolite of ketamine, or a prodrug thereof, or pharmaceutically acceptable salts thereof, are present in crystalline form.

In preferred embodiments of the first aspect of the present invention, the solid oral dosage form is selected from a tablet and a capsule.

In a second aspect of the present invention, the solid oral dosage forms of the first aspect comprise a blend of one or more diluent. Preferably the diluent blend comprises one or more, and preferably two or more, diluents selected from anhydrous lactose, D-mannitol, dicalcium phosphate, calcium carbonate, magnesium oxide, magnesium carbonate, glucose, sorbitol, sucrose, calcium sulphate, starch, dextrates, kaolinite, maltodextrin and lactitol. More preferred diluents are selected from microcrystalline cellulose, dicalcium phosphate, kaolinite, starch and calcium carbonate.

In preferred embodiments of the second aspect the solid oral dosage form is a tablet and comprises a diluent blend comprising microcrystalline cellulose. In further embodiments of the second aspect, the diluent blend comprises two or more diluents wherein one diluent is selected from microcrystalline cellulose and starch, and wherein a further diluent is an inorganic diluent.

In further embodiments of the second aspect, the diluent blend comprises dicalcium phosphate and microcrystalline cellulose.

In a preferred embodiment of the second aspect, the diluent blend excludes lactose monohydrate.

Preferred embodiments of the second aspect are wherein the solid oral dosage form is a capsule wherein the capsule shell comprises a constituent selected from gelatin and hydroxypropyl methylcellulose.

In preferred embodiments of the first aspect, the serotonin modulator is a selective serotonin reuptake inhibitor. Preferred serotonin modulators for use in the first aspect of the present invention are selected from vortioxetine, etoperidone, lorpiprazole, lubazodone, mepiprazole, nefazodone, trazodone, citalopram, escitalopram, fluoxetine, fluvoxamine, paroxetine, sertraline, dapoxetine, venlafaxine, milnacipran, duloxetine, levomilnacipran, desvenlafaxine, sibutramine, aptazapine, esmirtazapine, mianserin, mirtazapine, and setiptiline. Particularly preferred serotonin modulators for use in the first aspect of the present invention are selected from citalopram, escitalopram, fluoxetine, fluvoxamine, paroxetine, sertraline and dapoxetine.

A third aspect of the present invention provides the use of the solid oral dosage form in combination with a serotonin modulator, according to the first aspect, wherein the patient has experienced one or more manic episode or hypomanic episode, and/or is suffering or is at risk of suffering one or more manic episode or hypomanic episode.

In preferred embodiments of the aspects of the present invention, and in particular the third aspect, said patient is suffering from a condition selected from major depression, bipolar disorder Type I, bipolar disorder Type II, bipolar depression, postpartum depression, dementia-related depression, obsessive-compulsive disorder (OCD) with co-morbid depression, and post-traumatic stress disorder (PTSD) with co-morbid depression, tics with comorbid depression, and social anxiety with comorbid depression.

Prodrugs of ketamine are shown in Tables 8A and 8B and particularly preferred prodrugs for the solid oral dosage form are compounds numbered 5, 13, 21, 29, 37 and 45 in Tables 8A and 8B.

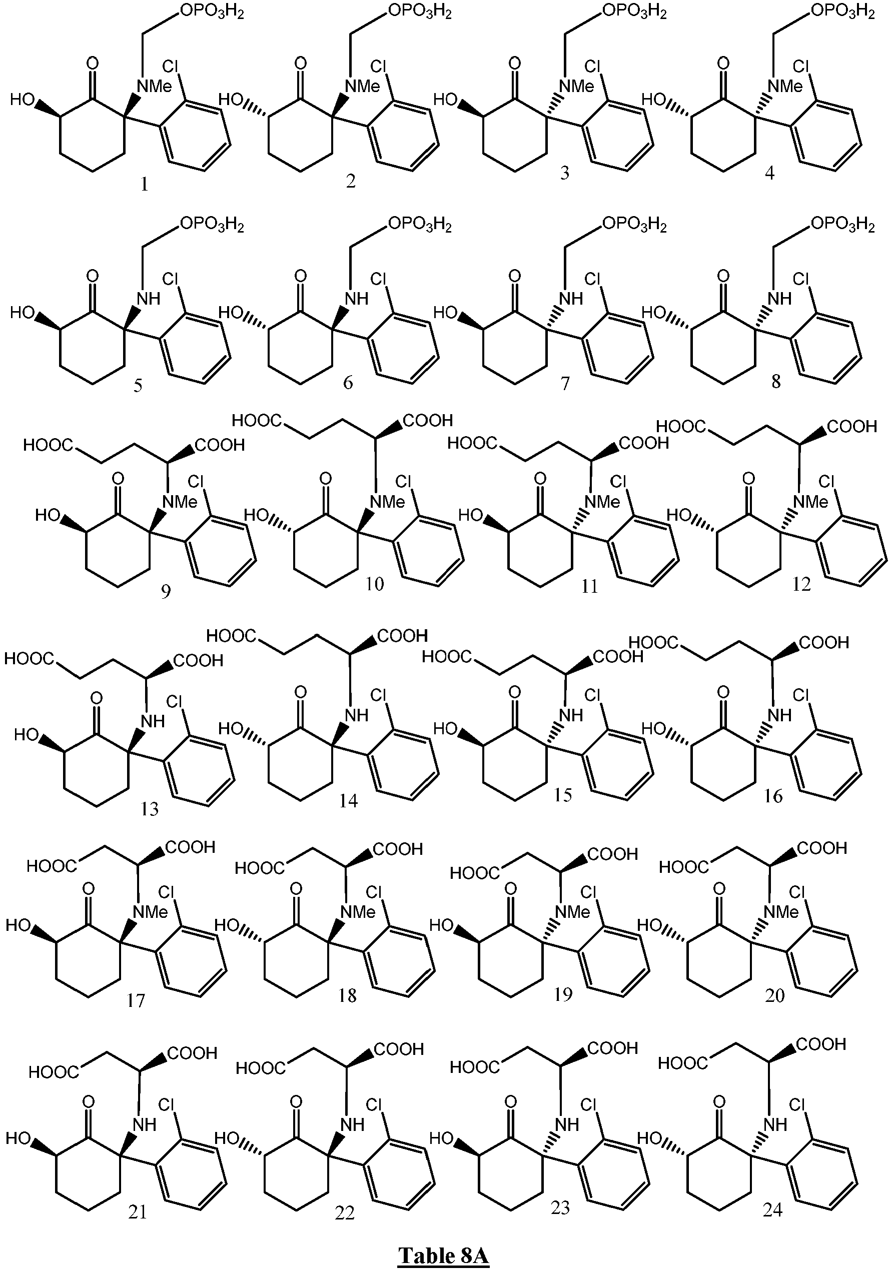

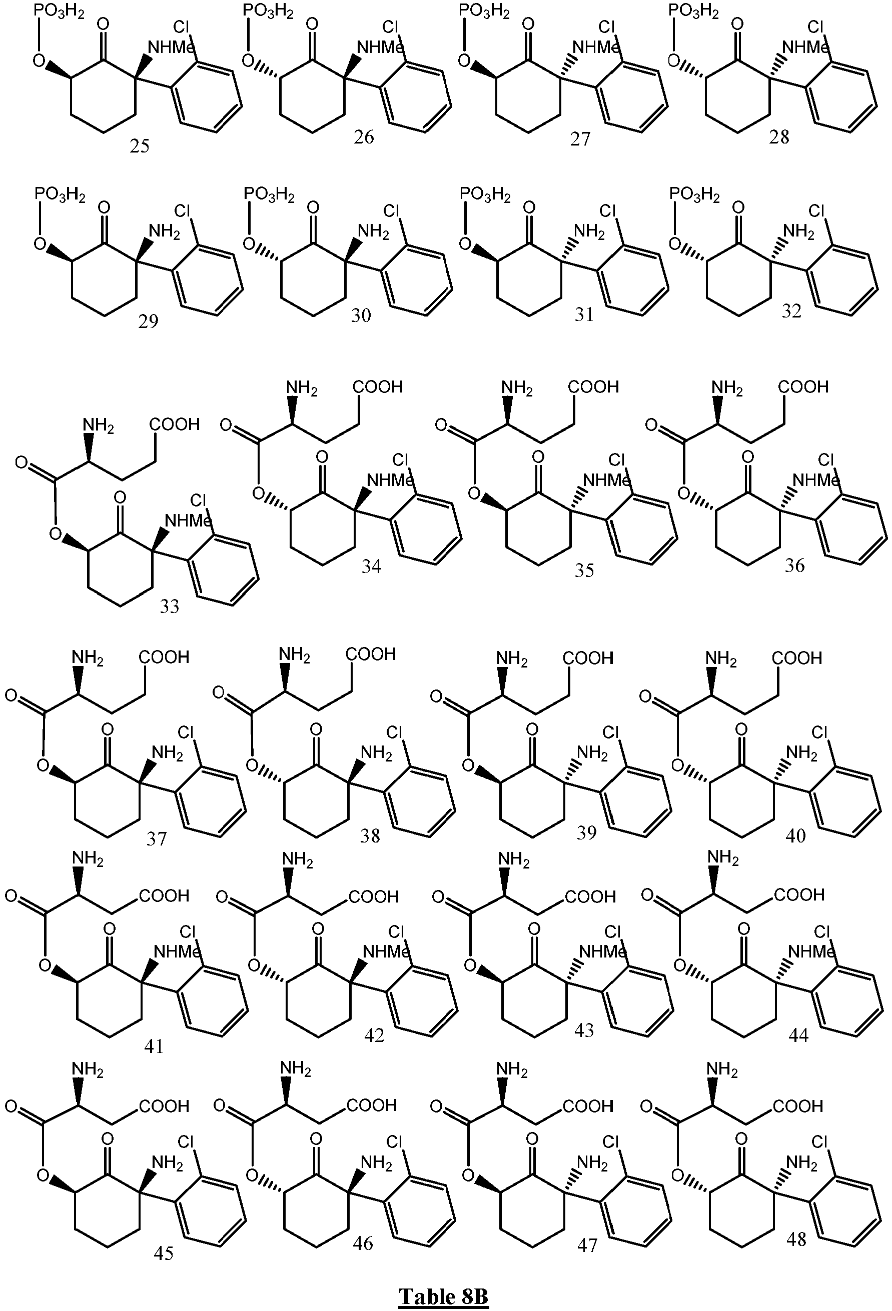

In a preferred embodiment of the first aspect of the present invention, the solid oral dosage form is for use in a patient who has failed to achieve adequate control of depression symptoms using psychotherapy alone.

In a preferred embodiment of the first aspect of the present invention, the solid oral dosage form is for use in a patient who has not previously been treated with an antidepressant agent.

In a fourth aspect of the present invention, the solid oral dosage form according to the aspects of the present invention comprises a tartrate salt of the seventh aspect of 2R,6R-hydroxynorketamine. Preferably the solid oral dosage form is selected from a tablet and a capsule. Preferably the tartrate salt is 2R,6R-hydroxynorketamine L(+)-tartrate.

In preferred embodiments of the fourth aspect of the present invention the solid oral dosage form comprises the active ingredient 2R,6R-hydroxynorketamine L-(+)-tartrate, wherein the active ingredient is present in an amount equivalent to between 10mg and 100mg of 2R,6R-hydroxynorketamine freebase.

In preferred embodiments of the fourth aspect of the present invention, the solid oral dosage forms comprise a blend of one or more diluent. Preferably the diluent blend comprises one or more, and preferably two or more, diluents selected from anhydrous lactose, D-mannitol, dicalcium phosphate, calcium carbonate, magnesium oxide, magnesium carbonate, glucose, sorbitol, sucrose, calcium sulphate, starch, dextrates, kaolinite, maltodextrin and lactitol. More preferred diluents are selected from microcrystalline cellulose, dicalcium phosphate, kaolinite, starch and calcium carbonate.

In preferred embodiments of the fourth aspect the solid oral dosage form is a tablet and comprises a diluent blend comprising microcrystalline cellulose. In further embodiments of the fourth aspect, the diluent blend comprises two or more diluents wherein one diluent is selected from microcrystalline cellulose and starch, and wherein a further diluent is an inorganic diluent.

In further embodiments of the fourth aspect, the diluent blend comprises dicalcium phosphate and microcrystalline cellulose.

In a preferred embodiment of the fourth aspect, the diluent blend excludes lactose monohydrate.

Preferred embodiments of the fourth aspect are wherein the solid oral dosage form is a capsule wherein the capsule shell comprises a constituent selected from gelatin and hydroxypropyl methylcellulose.

### BRIEF DESCRIPTION OF THE FIGURES

**Figure 1****:** Shows differential scanning calorimetry (DSC) analysis of a binary mix of 2R,6R-hydroxynorketamine and lactose monohydrate, in a 1:2 ratio of API:Excipient.
**Figure 2****:** Mean unbound fraction of 2R,6R-hydroxynorketamine in mouse, rat and human plasma.
**Figure 3****:** Shows the degree of UGT inhibition by 2R,6R-hydroxynorketamine in *in vitro* assays.
**Figure 4****:** Shows the rate of hepatic clearance in human, rat and dog hepatocyte assays.

### DETAILED DESCRIPTION OF THE INVENTION

The present invention is set out in the appended claims. Any embodiment not falling under the scope of the appended claims does not form part of the invention.

Throughout this specification, one or more aspect of the invention may be combined with one or more features described in the specification to define distinct embodiments of the invention.

References herein to a singular of a noun encompass the plural of the noun, and vice-versa, unless the context implies otherwise.

As used herein, the term 'patient' preferably refers to a human patient, but may also refer to a domestic mammal. The term does not encompass laboratory mammals.

As used herein, the term 'first-line therapy' is defined as the first course of pharmaceutical treatment administered in response to an episode of a disorder. The term 'episode' refers to a single noteworthy happening in the course of a longer series of events, such as one critical period of several during a prolonged disorder.

As used herein, the term 'depressive disorder' is defined as a category of mental disorders characterized by at least one of: (a) depressed mood, such as feeling sad, empty or tearful, and (b) significantly reduced interest or feeling no pleasure in all or most activities over a two-week period, combined with at least one further symptom selected from: (c) significant weight loss when not dieting, weight gain, or decrease or increase in appetite, (d) insomnia or increased desire to sleep (e) either restlessness or slowed behaviour that can be observed by others (f) fatigue or loss of energy, (g) feelings of worthlessness, or excessive or inappropriate guilt, (h) trouble making decisions, or trouble thinking or concentrating, and (i) recurrent thoughts of death or suicide, or a suicide attempt. Depressive disorders include: major depression, persistent depressive disorder, bipolar disorder, psychotic depression, postpartum depression, premenstrual dysphoric disorder (PMDD) and atypical depression.

Major depression, also referred to as clinical depression, is defined as the presence of five or more of the following symptoms over a period of two-weeks or more (also referred to herein as a 'major depressive episode'), most of the day, nearly every day.
- depressed mood, such as feeling sad, empty or tearful (in children and teens, depressed mood can appear as constant irritability);
- significantly reduced interest or feeling no pleasure in all or most activities;
- significant weight loss when not dieting, weight gain, or decrease or increase in appetite (in children, failure to gain weight as expected);
- insomnia or increased desire to sleep;
- either restlessness or slowed behaviour that can be observed by others;
- fatigue or loss of energy;
- feelings of worthlessness, or excessive or inappropriate guilt;
- trouble making decisions, or trouble thinking or concentrating;
- recurrent thoughts of death or suicide, or a suicide attempt.

At least one of the symptoms must be either a depressed mood or a loss of interest or pleasure.

Persistent depressive disorder, also known as dysthymia, is defined as a patient exhibiting the following two features:
A. has depressed mood for most the time almost every day for at least two years. Children and adolescents may have irritable mood, and the time frame is at least one year.
B. While depressed, a person experiences at least two of the following symptoms:
   - Either overeating or lack of appetite.
   - Sleeping too much or having difficulty sleeping.
   - Fatigue, lack of energy.
   - Poor self-esteem.
   - Difficulty with concentration or decision making.
   - Feeling hopeless.

Bipolar disorder, also known as manic-depressive illness, is a disorder that causes unusual shifts in mood, energy, activity levels, and the ability to carry out day-to-day tasks.

There are three defined sub-categories of bipolar disorder; all of them involve clear changes in mood, energy, and activity levels. These moods range from periods of extremely "up," elated, and energized behaviour (known as manic episodes, and defined further below) to very sad, "down," or hopeless periods (known as depressive episodes). Less severe manic periods are known as hypomanic episodes.

Bipolar I Disorder- defined by manic episodes that last at least 7 days, or by manic symptoms that are so severe that the person needs immediate hospital care. Usually, depressive episodes occur as well, typically lasting at least 2 weeks. Episodes of depression with mixed features (having depression and manic symptoms at the same time) are also possible.

Bipolar II Disorder- defined by a pattern of depressive episodes and hypomanic episodes, but not the full-blown manic episodes described above.

Bipolar depression is defined as an individual who is experiencing depressive symptoms with a previous or coexisting episode of manic symptoms, but does not fit the clinical criteria for bipolar disorder.

Cyclothymic Disorder (also called cyclothymia)- defined by numerous periods of hypomanic symptoms as well as numerous periods of depressive symptoms lasting for at least 2 years (1 year in children and adolescents). However, the symptoms do not meet the diagnostic requirements for a hypomanic episode and a depressive episode.

Psychotic depression, also known as depressive psychosis, is defined as a major depressive episode that is accompanied by psychotic symptoms including hallucinations and delusions.

Postpartum depression, also referred to as postnatal depression, as referred to herein refers to a diagnosis that meets the criteria for major depression when occurring in a mother within the first year from giving birth.

Premenstrual dysphoric disorder as referred to herein, is present in a patient who meets the following criteria A-C.

**Criterion** A is that in most menstrual cycles during the past year, at least 5 of the following 11 symptoms (including at least 1 of the 4 listed) must be present in the final week before the onset of menses, start to improve within a few days after the onset of menses, and become minimal or absent in the week postmenses.
1. Marked lability (e.g., mood swings)
2. Marked irritability or anger
3. Markedly depressed mood
4. Marked anxiety and tension
5. Decreased interest in usual activities
6. Difficulty in concentration
7. Lethargy and marked lack of energy
8. Marked change in appetite (e.g., overeating or specific food cravings)
9. Hypersomnia or insomnia
10. Feeling overwhelmed or out of control
11. Physical symptoms (e.g., breast tenderness or swelling, joint or muscle pain, a sensation of 'bloating' and weight gain)

**Criterion B one** (or more) of the following symptoms must be present:
1. Marked affective lability (e.g., mood swings; feeling suddenly sad or tearful, or increased sensitivity to rejection).
2. Marked irritability or anger or increased interpersonal conflicts.
3. Marked depressed mood, feelings of hopelessness, or self-deprecating thoughts.
4. Marked anxiety, tension, and/or feelings of being keyed up or on edge.

**Criterion C** one (or more) of the following symptoms must be additionally be present, to reach a total of *five* symptoms when combined with symptoms from Criterion B above.
1. Decreased interest in usual activities (e.g., work, school, friends, hobbies).
2. Subjective difficulty in concentration.
3. Lethargy, easy fatigability, or marked lack of energy.
4. Marked change in appetite; overeating; or specific food cravings.
5. Hypersomnia or insomnia.
6. A sense of being overwhelmed or out of control.
7. Physical symptoms such as breast tenderness or swelling, joint or muscle pain, a sensation of "bloating," or weight gain.

The symptoms in Criteria A-C must have been met for most menstrual cycles that occurred in the preceding year.

Atypical depression is a subtype of major depression or dysthymic disorder that involves several specific symptoms, including increased appetite or weight gain, sleepiness or excessive sleep, marked fatigue or weakness, moods that are strongly reactive to environmental circumstances, and feeling extremely sensitive to rejection.

Post-traumatic stress disorder (PTSD) is a condition which develops following a stressful event or situation of an exceptionally threatening or catastrophic nature, which is likely to cause pervasive distress in almost anyone. PTSD does not therefore develop following those upsetting situations that are described as 'traumatic' in everyday language, for example, divorce. PTSD symptoms include re-experiencing, such as flashbacks, avoidance of reminders of the trauma or rumination and hyperarousal or emotional numbing. PTSD with co-morbid depression is defined as PTSD in an individual who also meets the criteria for one or more depressive disorder.

Obsessive-compulsive disorder (OCD) is defined by the presence of either obsessions or compulsions, but commonly both. The symptoms can cause significant functional impairment and/or distress. An obsession is defined as an unwanted intrusive thought, image or urge that repeatedly enters the person's mind. Compulsions are repetitive behaviours or mental acts that the person feels driven to perform. A compulsion can either be overt and observable by others, such as checking that a door is locked, or a covert mental act that cannot be observed, such as repeating a certain phrase in one's mind. OCD with co-morbid depression is defined as OCD in an individual who also meets the criteria for one or more depressive disorder.

Social anxiety disorder is defined as the persistent fear and anxiety about one or more social or performance situations. The two main forms are generalised social anxiety w performance social anxiety. Generalised social anxiety affects most, if not all areas of life. This is the more common type and affects around 70% of those affected by the disorder. Performance social anxiety is where these feelings only occur in a few specific situations such as public speaking, eating in public or dealing with figures of authority. Social anxiety disorder with co-morbid depression is defined as social anxiety disorder in an individual who also meets the criteria for one or more depressive disorder.

Tic disorders are defined as neuropsychiatric syndrome, characterised by motor and vocal tics, which runs a fluctuating course. Tics can be defined as sudden, purposeless, repetitive, non-rhythmic, stereotyped movements or vocalisations for example eye twitching or blinking. Examples of vocal tics are throat clearing, grunting and barking. Tic disorder with co-morbid depression is defined as social anxiety disorder in an individual who also meets the criteria for one or more depressive disorder.

As used herein, the term 'ketamine' refers to 2-(2-Chlorophenyl)-2-(methylamino)-cyclohexanone. As used herein, the term '2R,6R-hydroxynorketamine' refers to 2R,6R-2-(2-Chlorophenyl)-2-(amino)-6-hydroxycyclohexanone.

As used herein, the term 'metabolite of ketamine' includes R-norketamine, S-norketamine, 2R,6R-hydroxyketamine, 2R,6S-hydroxyketamine, 2S,6R-hydroxyketamine, 2S,6S-hydroxyketamine, R-(5,6)dehydroketamine, S-(5,6)dehydroketamine, R-(3,4)dehydroketamine, S-(3,4)dehydroketamine, R-(5,6)dehydronorketamine, S-(5,6)dehydronorketamine, R-(3,4)dehydronorketamine, S-(3,4)dehydronorketamine, 2R,6R-hydroxynorketamine, 2R,6S-hydroxynorketamine, 2S,6R-hydroxynorketamine, 2S,6S-hydroxynorketamine, 2R,5R-hydroxynorketamine, 2R,5S-hydroxynorketamine, 2S,5R-hydroxynorketamine, 2S,5S-hydroxynorketamine, 2R,6S-hydroxynorketamine, 2R,4S-hydroxynorketamine, 2S,4R-hydroxynorketamine, 2S,4S-hydroxynorketamine or any mixture thereof, including any racemic mixture thereof, or any pharmaceutically acceptable salt thereof.

As used herein, the term 'prodrug of a metabolite of ketamine' is defined as a compound formed by the covalent bonding of the amine group or hydroxyl group of a metabolite of ketamine with a biolabile moiety, or 'promoiety', as defined by Formula Ib.

As used herein, the term 'pharmaceutically acceptable salt' is defined as the product of a reaction with an acid or base to form a non-toxic salt. The term pharmaceutically acceptable salt includes solvates thereof. Wherever a compound is referred to by its generic or systematic name, the term is taken to include all pharmaceutically acceptable salts.

In a preferred embodiment, the combination of the invention is for treating a depressive disorder selected from the list comprising major depression, persistent depressive disorder, bipolar disorder (type 1 or type 2), psychotic depression, postpartum depression, premenstrual dysphoric disorder (PMDD), atypical depression, post-traumatic stress disorder (PTSD) with comorbid depression, obsessive compulsive disorder (OCD) with comorbid depression, social anxiety with co-morbid depression, and tic disorders (eg. Tourette's Syndrome) with co-morbid depression.

In a preferred embodiment, the depressive disorder is major depression.

In an alternative preferred embodiment, the depressive disorder is persistent depressive disorder. In an alternative preferred embodiment the depressive disorder is bipolar disorder.

In a preferred embodiment, the depressive disorder is selected from bipolar depression, postpartum depression, premenstrual dysphoric disorder (PMDD), atypical depression, post-traumatic stress disorder (PTSD) with comorbid depression, obsessive compulsive disorder (OCD) with comorbid depression, social anxiety with co-morbid depression, and tic disorders (eg. Tourette's Syndrome) with co-morbid depression.

### Prodrugs

Prodrugs of 2R,6R-hydroxynorketamine can be synthesized by analogy from the corresponding metabolite of ketamine or analogue thereof via the synthesis described in Scheme 1:

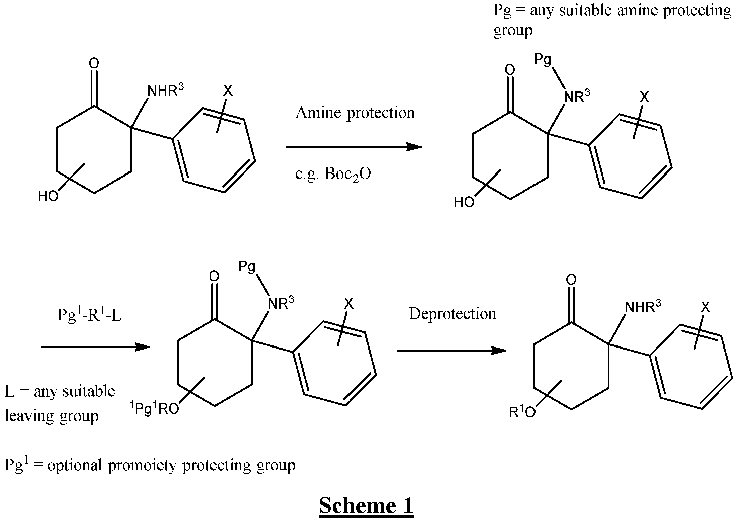

In particular, O-phosphate prodrugs of ketamine metabolites may be synthesized from their corresponding ketamine metabolite via the synthesis provided in Scheme 2:

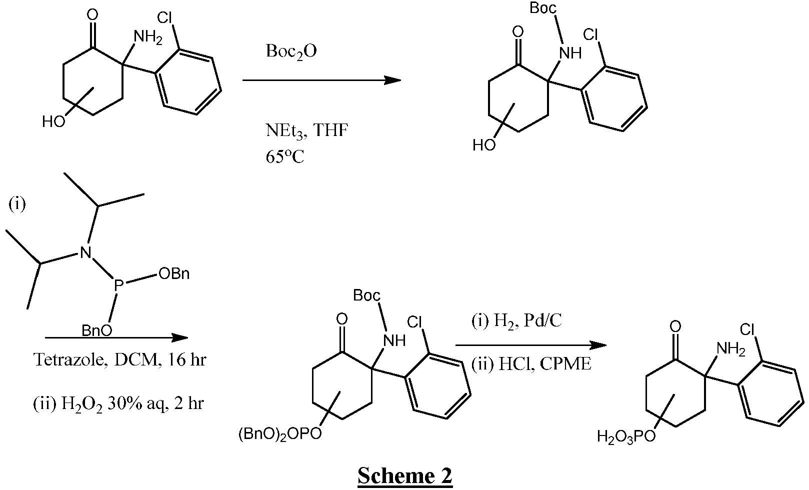

Prodrugs with amine-bonded promoieties can be synthesized from the corresponding metabolite of ketamine or analogue thereof via the synthesis described in Scheme 3:

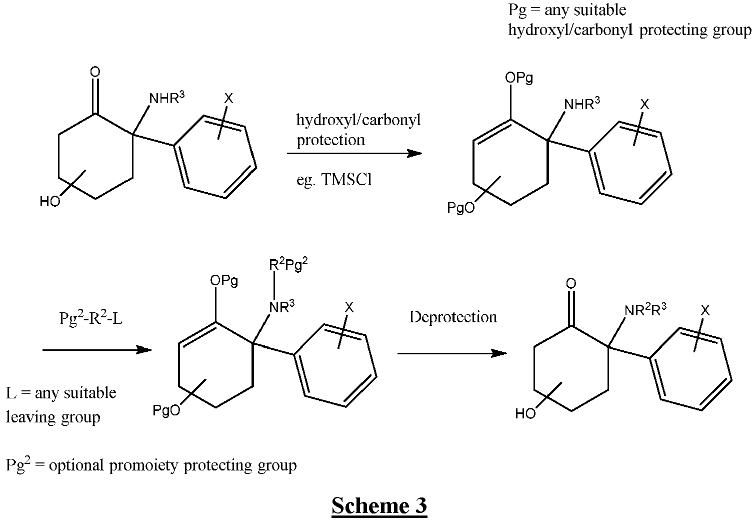

In particular, N-methylenephosphate prodrugs of ketamine metabolites may be synthesized from their corresponding ketamine metabolite via the synthesis provided in Scheme 4:

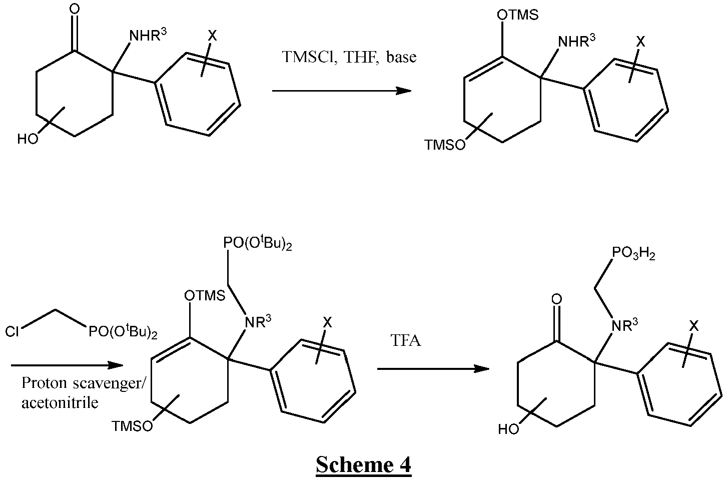

The prodrug of 2R,6R-hydroxynorketamine according to the present invention has a structure of Formula Ib, or a pharmaceutically acceptable salt thereof, 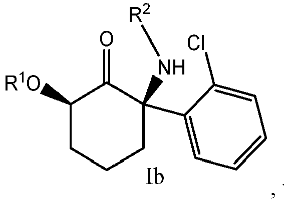 wherein R¹ and R² are as defined in claim 1.

In preferable prodrugs of Formula Ib, R¹ is selected from PO₃H₂, SO₃H, CO₂H, and R⁵, wherein R⁵ is selected from 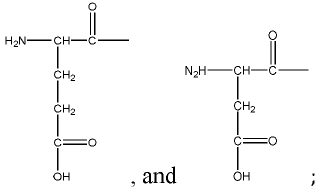 and
R² is H.

In another embodiment, R¹ is H; and
R² is selected from CH₂OPO₃H₂, CH₂OSO₃H and R⁴, wherein R⁴ is selected from the groups listed in Table 1;

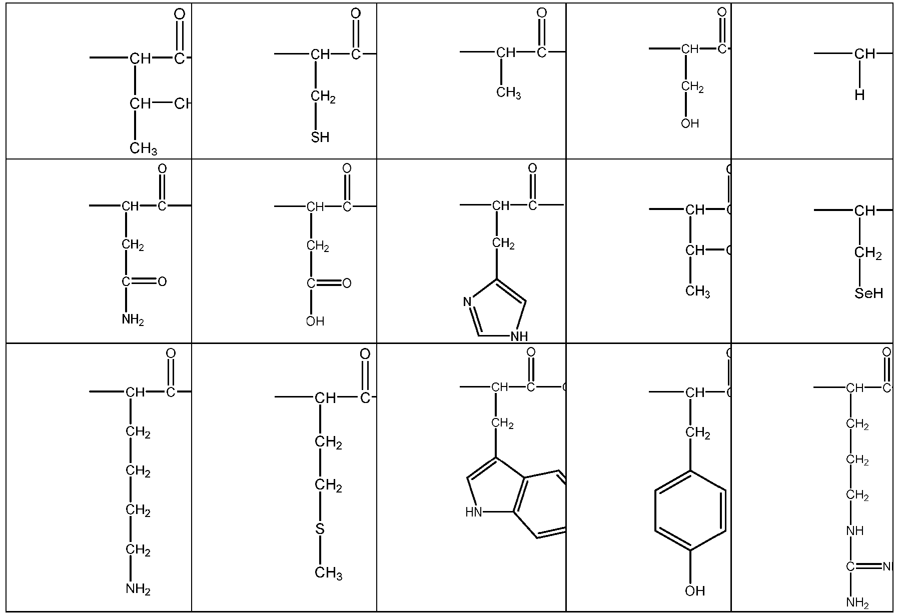

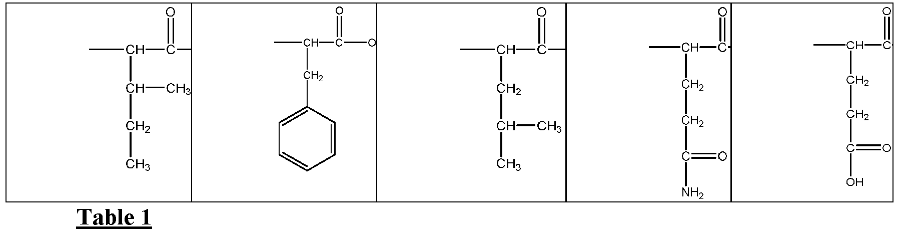

### Tartrate salt of metabolites of ketamine

An aspect of the present invention provides tartrate salts of the metabolite of ketamine as described herein. It has been discovered that tartrate salts of metabolites of the present invention are suitable for use in oral formulations of the present invention. A further advantage of using tartrate salts in the solid dosage forms of the present invention is that either L-(+)-tartaric acid or D-(-)-tartaric acid may be employed in chiral recrystallization to aid separation of the metabolite of ketamine from its enantiomer. Thus, the appropriate tartrate salt of metabolites of ketamine described herein may be manufactured into solid oral dosage forms directly following chiral separation from its enantiomers and/or diastereomers.

A preferred embodiment of the present invention is a solid oral dosage form comprising a tartrate salt of the metabolite of ketamine or a prodrug of a metabolite of ketamine, wherein the solid oral dosage form is for administration in combination with a serotonin modulator for use in the treatment of a depressive disorder in a patient. A particularly preferred embodiment of this is when the salt of the metabolite is 2R,6R-hydroxynorketamine L-(+)-tartrate.

In preferred embodiments of the present invention the pharmaceutically acceptable salt comprises 2R,6R-hydroxynorketamine wherein the tartrate salt comprises L-(+)-tartrate, preferably wherein 80% or more of the metabolite of ketamine is 2R,6R-hydroxynorketamine.

In preferred embodiments of the invention, the tartrate salt of the metabolite of ketamine is crystalline.

In embodiments, the solid oral dosage form of the present invention comprises a tartrate salt. Preferably the solid oral dosage form is selected from tablet and capsule. Preferably the tartrate salt is 2R,6R-hydroxynorketamine L(+)-tartrate.

In preferred embodiments the solid oral dosage form comprises the active ingredient 2R,6R-hydroxynorketamine L-(+)-tartrate, wherein the active ingredient is present in an amount equivalent to between 10mg and 100mg of 2R,6R-hydroxynorketamine freebase.

In preferred embodiments of solid oral dosage forms comprising tartrate salts of the present invention, the solid oral dosage forms comprise a blend of one or more diluents. Preferably the diluent blend comprises one or more, and preferably two or more, diluents selected from anhydrous lactose, D-mannitol, dicalcium phosphate, calcium carbonate, magnesium oxide, magnesium carbonate, glucose, sorbitol, sucrose, calcium sulphate, starch, dextrates, kaolinite, maltodextrin and lactitol. More preferred diluents are selected from microcrystalline cellulose, dicalcium phosphate, kaolinite, starch and calcium carbonate.

In preferred embodiments of solid oral dosage forms comprising tartrate salts of the present invention, the solid oral dosage form is a tablet and comprises a diluent blend comprising microcrystalline cellulose. In further embodiments the diluent blend comprises two or more diluents wherein one diluent is selected from microcrystalline cellulose and starch, and wherein a further diluent is an inorganic diluent.

Preferred embodiments of solid oral dosage forms comprising tartrate salts of the present invention comprise dicalcium phosphate and microcrystalline cellulose.

In preferred embodiments of solid oral dosage forms comprising tartrate salts of the present invention, solid oral dosage form comprises a diluent blend which excludes lactose monohydrate.

Preferred embodiments of solid oral dosage forms comprising tartrate salts of the present invention, the solid oral dosage form is a capsule wherein the capsule shell comprises a constituent selected from gelatin and hydroxypropyl methylcellulose.

### Combination with serotonin modulators

The present invention provides a metabolite of ketamine or a pharmaceutically acceptable salt thereof, or a prodrug thereof, for co-administration with a serotonin modulator. As used herein, the term 'serotonin modulator' is defined as any compound which affects the serotonin neurotransmitter (serotonergic system). Serotonin modulators include serotonin stimulators (e.g. vortioxetine), serotonin antagonist and reuptake inhibitors (SARIs; e.g. etoperidone, lorpiprazole, lubazodone, mepiprazole, nefazodone, and trazodone), selective serotonin reuptake inhibitors (SSRIs; e.g. citalopram, escitalopram, fluoxetine, fluvoxamine, paroxetine, sertraline, dapoxetine), serotonin-norepinephrine reuptake inhibitors (SNRIs; e.g. venlafaxine, milnacipran, duloxetine, levomilnacipran, desvenlafaxine, sibutramine), and noradrenergic and specific serotonergic antidepressants (NaSSAs; e.g. aptazapine, esmirtazapine, mianserin, mirtazapine, setiptiline).

As used herein, the term 'coadministration' is defined as either the administration of a formulation which contains both the dosage form of the present invention and the serotonin modulator, or the simultaneous, essentially simultaneous, sequential or separate administration within a given dosing period of separate formulations containing the solid oral dosage form of the present invention and the serotonin modulator, respectively.

A problem common to all combination therapies is the risk of drug-drug interactions (DDIs) which can lead to unwanted side effects which are not present in one or other members of the combination. Specifically, the potential for adverse DDIs increases if one member of the combination affects the rate of metabolism of another member of the combination. Furthermore, when combined with a serotonin modulator, a drug which interferes with the serotonergic system may result in adverse DDIs. It has been discovered that ketamine metabolites according to the present invention do not adversely affect the rate of metabolism of serotonin modulators, nor do they interfere with the serotonergic system, and combinations of compounds of the present invention with serotonin modulators exhibit limited adverse DDIs.

A preferred class of serotonin modulators for use according to the present invention are SSRIs. Preferred SSRIs are citalopram, escitalopram, fluoxetine, fluvoxamine, paroxetine, sertraline and dapoxetine. Most preferred SSRIs are citalopram or escitalopram.

Accordingly, an aspect of the present invention provides a combination of an SSRI and the solid oral dosage form as described herein for use in the treatment of a depressive disorder in a patient. In a preferred embodiment, said combination is selected from the following list:
2R,6R-hydroxynorketamine and citalopram, 2R,6R-hydroxynorketamine and escitalopram, 2R,6R-hydroxynorketamine and fluoxetine, 2R,6R-hydroxynorketamine and fluvoxamine, 2R,6R-hydroxynorketamine and paroxetine, 2R,6R-hydroxynorketamine and sertraline, 2R,6R-hydroxynorketamine and dapoxetine,.

More preferred combinations according to the invention are selected from the following: 2R,6R-hydroxynorketamine and citalopram, 2R,6R-hydroxynorketamine and escitalopram. A particularly preferred combination according to the present invention is 2R,6R-hydroxynorketamine and escitalopram.

In a preferred embodiment of the invention, the compound is for use in a patient who has not previously been treated with an antidepressant agent. As used herein, the term 'antidepressant agent' is defined as any pharmaceutical product that is administered to combat or reduce the symptoms of a depressive disorder.

In a preferred embodiment of the invention, the patient is a patient who has failed to achieve adequate control of depression symptoms using psychotherapy alone. As used herein, the term 'depression symptoms' include:
- depressed mood, such as feeling sad, empty or tearful (in children and teens, depressed mood can appear as constant irritability);
- significantly reduced interest or feeling no pleasure in all or most activities;
- significant weight loss when not dieting, weight gain, or decrease or increase in appetite (in children, failure to gain weight as expected);
- insomnia or increased desire to sleep;
- either restlessness or slowed behaviour that can be observed by others;
- fatigue or loss of energy;
- feelings of worthlessness, or excessive or inappropriate guilt;
- trouble making decisions, or trouble thinking or concentrating;
- recurrent thoughts of death or suicide, or a suicide attempt;

As used herein, the term 'psychotherapy' is defined as the use of psychological methods to help a person change and overcome problems in desired ways.

Depression symptoms may be quantified using a Patient Health Questionnaire-9 (PHQ-9).

The PHQ-9 is a self-administered patient questionnaire which asks the patient the following question:
'Over the last two weeks, how often have you been bothered by any of the following problems?'
- Little interest or pleasure in doing things?
- Feeling down, depressed, or hopeless?
- Trouble falling or staying asleep, or sleeping too much?
- Feeling tired or having little energy?
- Poor appetite or overeating?
- Feeling bad about yourself - or that you are a failure or have let yourself or your family down?
- Trouble concentrating on things, such as reading the newspaper or watching television?
- Moving or speaking so slowly that other people could have noticed?
- Or the opposite - being so fidgety or restless that you have been moving around a lot more than usual?
- Thoughts that you would be better off dead, or of hurting yourself in some way?

The PHQ-9 score is calculated by assigning scores of 0, 1, 2, and 3, respectively, to the response categories of 'not at all', 'several days', 'more than half the days', and 'nearly every day', and adding together the scores for the nine questions.

In embodiments of the invention, a reduction in PHQ-9 score is achieved of 2 points or more, or 3 points or more, or 5 points or more, or 10 points or more, or 15 points or more, or 20 points or more over the period of a course. As used herein, the term 'course' or 'course of treatment' is the period of time in which the patient is treated in accordance with the present invention. In embodiments of the invention, a course is between 2 weeks and 12 months, or between 3 weeks and 6 months, or between 4 weeks and 5 months or between 6 weeks and 4 months, or between 2 months and 3 months. In a preferred embodiment of the invention, a course is 6 months or less. In a preferred embodiment of the invention, a course is three months or less.

Preferably, when used in combination with a serotonin modulator, the metabolite of the present invention may be administered in any formulation suitable for pharmaceutical administration. Preferably the metabolite of the present invention is in a dosage form selected from a tablet, a pill, a capsule, a caplet, a powder, granules, orodispersible tablet, sterile parenteral solution or suspension, powder for injection, metered aerosol or liquid spray, drops, ampoule, autoinjector, suppository, sublingual spray, sublingual patch, sublingual film, buccal patch, buccal spray, buccal film, intranasal sprayable solution, intranasal sprayable suspension, and intranasal powder. In preferred embodiments of the invention, the dosage form of the compound of the present invention is selected from orodispersible tablet, sublingual spray, sublingual patch, sublingual film, buccal patch, buccal spray, buccal film, intranasal sprayable solution, intranasal sprayable suspension, and intranasal powder, and is preferably a dosage form suitable for intranasal administration.

### Solid oral dosage forms

A solid oral dosage form is for use in the treatment of a depressive disorder in a patient.

Parenteral administration of metabolites of ketamine, in particular 2R,6R-hydroxynorketamine, have been found to be effective in treating depressive disorders in Zanos et al, Nature, (2016), 533, 481-486. However, these known parenteral formulations suffer some of the same drawbacks as are evident for the use of intravenous ketamine for treating depression, and are inappropriate for treating the majority of depressed patients, for whom treatment in an outpatient setting without the need of a medical professional would be preferable. There are, however, challenges in the development of alternative formulations which overcome the drawbacks of prior art formulations. For example, freebase metabolites of ketamine such as 2R,6R-hydroxynorketamine exist as a viscous oil under ambient conditions and are particularly difficult to process into a pharmaceutical formulation unless in the liquid state.

As used herein, the term 'solid oral dosage form' is defined as a solid pharmaceutical formulation which can be swallowed whole, chewed and swallowed, or dissolved, dispersed or absorbed via the oral cavity. Solid oral dosage forms include tablets, pills, capsules, caplets, orodispersible tablets, powders, granules and gums. Solid oral dosage forms are not taken to include liquid or aerosol formulations, powders for inhalation, or powders for injection.

In preferred embodiments of the first aspect of the present invention, the metabolite of ketamine is in the form of an acid-addition salt.

In a preferred embodiment, the solid oral dosage form for use according to the invention is for treating a depressive disorder selected from the list comprising major depression, persistent depressive disorder, bipolar disorder, psychotic depression, postpartum depression, premenstrual dysphoric disorder (PMDD) and atypical depression.

In a preferred embodiment, the solid oral dosage form is for use in treating major depression.

In a preferred embodiment, the solid oral dosage form is for use in treating persistent depressive disorder.

In a preferred embodiment, the solid oral dosage form is for use in treating a bipolar disorder.

In a preferred embodiment, the solid oral dosage form is for use in treating bipolar depression.

In a preferred embodiment, the solid oral dosage form is for use in treating postpartum depression.

In a preferred embodiment, the solid oral dosage form is for use in treating OCD with co-morbid depression.

In a preferred embodiment, the solid oral dosage form is for use in treating PTSD with co-morbid depression.

In a preferred embodiment, the solid oral dosage form is for use in treating a social anxiety disorder with co-morbid depression.

According to the invention, the solid oral dosage form further comprises a serotonin modulator. A preferred class of serotonin modulators comprised in the solid oral dosage form of the present invention are SSRIs.

In a preferred embodiment, the solid oral dosage form of the present invention is for use in a patient who has not previously been treated with an antidepressant agent. In a preferred embodiment of the invention, the solid oral dosage form is for use in a patient who has failed to achieve adequate control of anxiety symptoms using psychotherapy alone.

In preferred embodiments of the first aspect of the present invention, the solid oral dosage form comprises 2R,6R-hydroxynorketamine in the form of an acid-addition salt. It has been found that metabolites of ketamine of the present invention, and 2R,6R-hydroxynorketamine, present challenges for solid oral dosage formulation as a freebase, because they are viscous oils at ambient conditions. Accordingly, the formulation of solid oral dosage forms metabolites of ketamine according to the present invention are preferably prepared using an acid addition salt of said metabolite.

Solid oral dosage forms according to the present invention may be prepared by mixing the principle active agent(s) with a pharmaceutical carrier, e.g. corn starch, lactose, sucrose, sorbitol, talc, stearic acid, magnesium stearate, or dicalcium phosphate, and other pharmaceutical diluents, e.g. water, to form a solid preformulation composition containing a homogenous mixture of the active agent(s). When referring to these preformulation compositions as homogenous, it is meant that the active agent is dispersed evenly throughout the composition so that the composition may be readily subdivided into equally effective dosage forms such as tablets, pills and capsules. The solid preformulation composition is then subdivided into unit dosage forms of the type described above.

Suitable unit doses of the solid oral dosage form according to the present invention contain between 0.5mg and 2000mg of the metabolite of ketamine, or pharmaceutically acceptable salt thereof, or prodrug thereof, or between 1mg and 900mg, or between 2mg and 800mg, or between 3mg and 700mg, or between 4mg and 600mg, or between 5mg and 500mg, or between 6mg and 400mg, or between 7mg and 300mg, or between 8mg and 200mg, or between 9mg and 150mg, or between 10 mg and 100 mg, or between 20mg and 90mg, or between 30mg and 80mg, or between 40mg and 70mg, or between 50mg and 60mg. In preferred embodiments of the present invention, the solid oral dosage form is provided in a unit dose containing between 10 mg and 100 mg of the metabolite of ketamine, or pharmaceutically acceptable salt thereof, or prodrug thereof. In preferred embodiments of the present invention, the solid oral dosage form is provided in a unit dose containing between 10 mg and 100 mg of 2R,6R-hydroxynorketamine, or pharmaceutically acceptable salt thereof, or prodrug thereof.

Quantities of weight provided herein refer to the free-form equivalent of a compound of the present invention. For example a unit dose of 50mg 2R,6R-hydroxynorketamine hydrochloride, contains the mass equivalent of 50mg freebase 2R,6R-hydroxynorketamine, and has an actual mass of 57.6 mg.

Suitable unit doses of the serotonin modulators for use in aspects of the present invention include the unit doses for these compounds as described in Martindale: The Complete Drug Reference (London, the Pharmaceutical Press, 38th Edition) and US Pharmacopoeia and National Formulary 2016 Edition.

Suitable unit doses for citalopram include 10mg, 20mg and 40mg. Suitable unit doses for escitalopram include 5mg, 10mg and 20mg. Suitable unit doses for fluoxetine include 20mg and 60mg. Suitable unit doses for fluvoxamine include 50mg and 100mg. Suitable unit doses for paroxetine include 10mg, 20mg and 30mg. Suitable unit doses for sertraline include 50mg and 100mg. Suitable unit doses for dapoxetine include 30mg and 60mg.

Solid oral dosage forms according to the present invention are preferably formulated in unit doses for administration between once a day and once a week, or between once every two days and twice a week. In a preferred embodiment the unit dose is for administration once a day.

Preferred embodiments of the invention are solid oral dosage forms comprising 2R,6R-hydroxynorketamine or a pharmaceutically acceptable salt thereof.

Preferably the solid oral dosage form comprises between 10 mg and 100mg of 2R,6R-hydroxynorketamine, or the equivalent thereof.

Preferably the solid oral dosage form comprises a crystalline form of 2R,6R-hydroxynorketamine or a pharmaceutically acceptable salt thereof.

Preferably the solid oral dosage form comprises a pharmaceutically acceptable salt of 2R,6R-hydroxynorketamine with the proviso that the solid oral dosage form does not comprise 2R,6R-hydroxynorketamine hydrochloride.

Preferably the solid oral dosage form of 2R,6R-hydroxynorketamine or a pharmaceutically acceptable salt thereof is a capsule or a tablet.

Preferably the solid oral dosage form of 2R,6R-hydroxynorketamine or a pharmaceutically acceptable salt thereof comprises a blend of one or more diluent.

Preferably the solid oral dosage form of 2R,6R-hydroxynorketamine or a pharmaceutically acceptable salt thereof is a tablet wherein the blend of one or more diluent comprises microcrystalline cellulose.

Preferably the solid oral dosage form of 2R,6R-hydroxynorketamine or a pharmaceutically acceptable salt thereof comprises a blend of one or more diluent with the proviso that the dosage form does not contain lactose monohydrate.

Preferably the solid oral dosage form of 2R,6R-hydroxynorketamine or a pharmaceutically acceptable salt thereof is a capsule and the capsule shell comprises a constituent selected from gelatin and hydroxypropyl methylcellulose.

The serotonin modulator is preferably a selective serotonin reuptake inhibitor (SSRI), more preferably selected from Citalopram, Escitalopram, Paroxetine, Fluoxetine, Fluvoxamine, Sertraline, Desvenlafaxine, Duloxetine, Levomilnacipran, Milnacipran, Tofenacin, Venlafaxine, Vilazodone, Vortioxetine, Etoperidone, Nefazodone, and Trazodone, or a pharmaceutically acceptable salt thereof, and most preferably selected from Citalopram, Escitalopram, Paroxetine, Sertraline, Duloxetine, and Venlafaxine, or a pharmaceutically acceptable salt thereof.

Preferably the solid oral dosage form of 2R,6R-hydroxynorketamine or a pharmaceutically acceptable salt thereof and the serotonin modultator are for administration simultaneously, sequentially or separately.

In preferred embodiments of the present invention the solid oral dosage form of 2R,6R-hydroxynorketamine or a pharmaceutically acceptable salt thereof is for treating a patient wherein the patient has experienced one or more manic episode or hypomanic episode, or is suffering a manic episode or hypomanic episode, or is at risk of suffering one or more manic episode or hypomanic episode.

In preferred embodiments of the present invention the solid oral dosage form of 2R,6R-hydroxynorketamine or a pharmaceutically acceptable salt thereof is for use in treating a patient suffering from bipolar depression type 1, bipolar depression type 2, bipolar depression, or obsessive-compulsive disorder comorbid with depression.

According to the present invention the solid oral dosage form of 2R,6R-hydroxynorketamine or a pharmaceutically acceptable salt thereof is for use as a first-line therapy.

In preferred embodiments of the present invention the solid oral dosage form comprises 2R,6R-hydroxynorketamine L-(+)-tartrate.

### Patients at risk of mania

In a particularly preferred embodiment, the combination of claim 1 of the present invention is useful in the treatment of a depressive disorder in a patient who has experienced one or more manic episode or hypomanic episode. In an alternative embodiment, the present invention is for use in the treatment of a depressive disorder in a patient who is suffering or is at risk of suffering one or more manic episode or hypomanic episode. Such patients include, but are not limited to, patients suffering from major depressive disorder or bipolar disorder.

As used herein, the term 'manic episode' is defined as a distinct period of abnormally and persistently elevated, expansive, or irritable mood and abnormally and persistently increased activity or energy, lasting at least 1 week and present most of the day, nearly every day, or any duration if hospitalization is necessary. As used herein the term 'hypomanic episode' is defined as a mood state characterized by persistent disinhibition and pervasive elevated euphoria with or without irritable mood for a period of four days or more.

Ketamine induced mania has been reported (Ricke AK, Snook RJ, Anand A. Induction of prolonged mania during ketamine therapy for reflex sympathetic dystrophy. Biol Psychiatry. 2011;70(4):p13-14). It has now been discovered that 2R,6R-hydroxynorketamine exhibits neither dopamine receptor agonism, nor activity at dopamine transporters. Accordingly, an aspect of the present invention provides 2R,6R-hydroxynorketamine, or a pharmaceutically acceptable salt thereof, or a prodrug thereof, in combination with serotonin modulators described herein, for use in a patient who has experienced one or more manic episode or hypomanic episode, or in a patient who is suffering or at risk of suffering one or more manic episode or hypomanic episode.

### First-line Therapy

The present invention is for use as a first-line therapy.

Intravenous infusions of 0.5mg/kg of ketamine hydrochloride administered over a period of 40 mins are known to exhibit potent and rapid anti-depressant effects in treatment-resistant depression patients, reported, for example, in Murrough et al; Antidepressant Efficacy of Ketamine in Treatment-Resistant Major Depression: A Two-Site Randomized Controlled Trial; Am J Psychiatry. 1 October 2013; 170(10): 1134-1142.

However neither metabolites of ketamine, nor prodrugs of ketamine or its metabolites have been investigated as a first-line therapy for the treatment of a depressive disorder in a patient. The present invention provides, for the first time, a first-line therapy for use in the treatment of a patient suffering from a depressive disorder which overcomes drawbacks associated with existing first-line therapies.

Moreover, the use of ketamine hydrochloride in the treatment of mental health is limited by the dosage form in which it is typically provided. Ketamine hydrochloride is approved in a form administrable only by intravenous infusion. This renders it inconvenient and/or inappropriate for use as a first-line therapy: it is preferable to be able to administer first-line therapy in an out-patient setting. Accordingly, the present invention provides the first-line therapy as defined in claim 1.

### EXAMPLES

### Example 1: Formation of a crystalline solid form of 2R,6R-hydroxynorketamine

Racemic *cis*-hydroxynorketamine was synthesized according to the synthesis provided in Scheme 5.

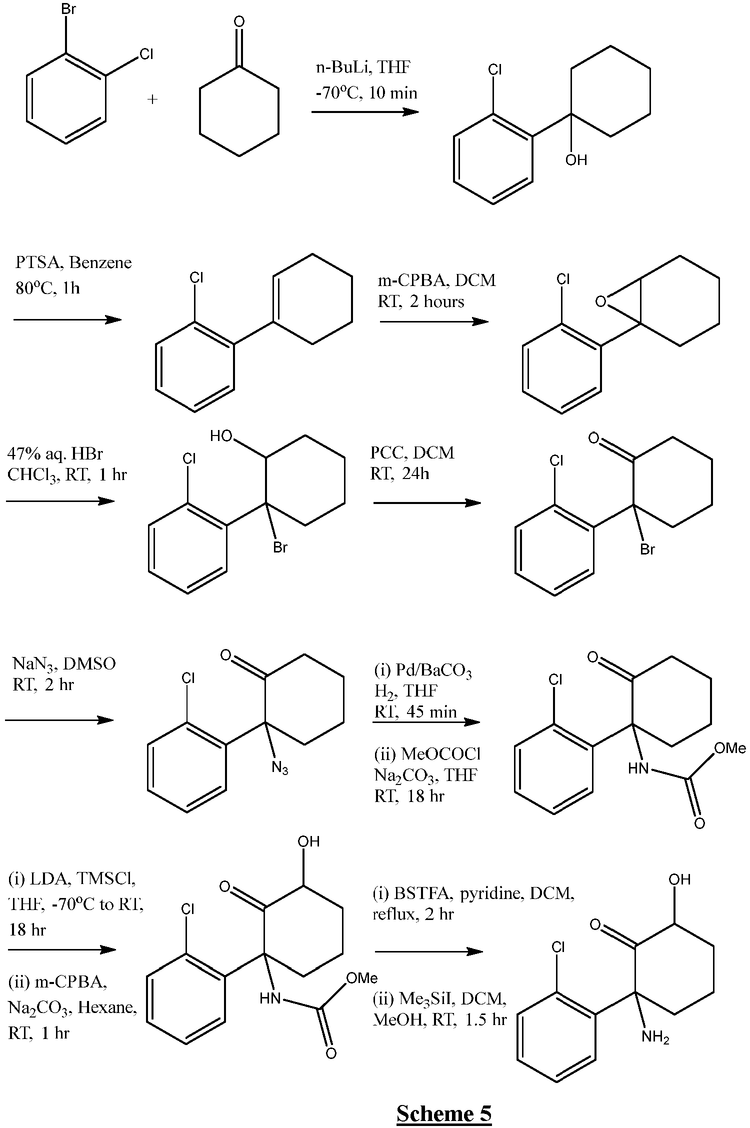

Crude obtained from Scheme 5 was purified by flash chromatography to produce *cis-*6-hydroxynorketamine freebase (racemic mixture of 2R,6R-hydroxynorketamine and 2S,6S-hydroxynorketamine). The racemic form of *cis*-6-hydroxynorketamine forms an amorphous oil at ambient conditions.

Racemic *cis*-hydroxynorketamine was dissolved to 25 mg/mL in methanol/IPA (1:1) and was then purified by SFC. Combined fractions of each of 2R,6R-hydroxynorketamine and 2S,6S-hydroxynorketamine were then evaporated to near dryness using a rotary evaporator, transferred into final vessels with DCM, which was removed under a stream of compressed air at 40C before being stored in a vacuum oven at 40C and 5mbar for 16 hours to afford each of 2R,6R-hydroxynorketamine and 2S,6S-hydroxynorketamine. Both 2R,6R-hydroxynorketamine and 2S,6S-hydroxynorketamine form an amorphous oil at ambient conditions.

In an alternative separation method, a crystalline form of 2R,6R-hydroxynorketamine L-(+)-tartrate was obtained by treating racemic cis-hydroxynorketamine freebase with L-(+)-tartaric acid in methanol followed by recrystallization from acetone.

### Example 2: Preparation of O-phosphate prodrug of 2R,6R-hydroxynorketamine

(1R,3R)-3-amino-3-(2-chlorophenyl)-2-oxocyclohexyl dihydrogen phosphate (CI) was prepared following the synthesis of Scheme 6.

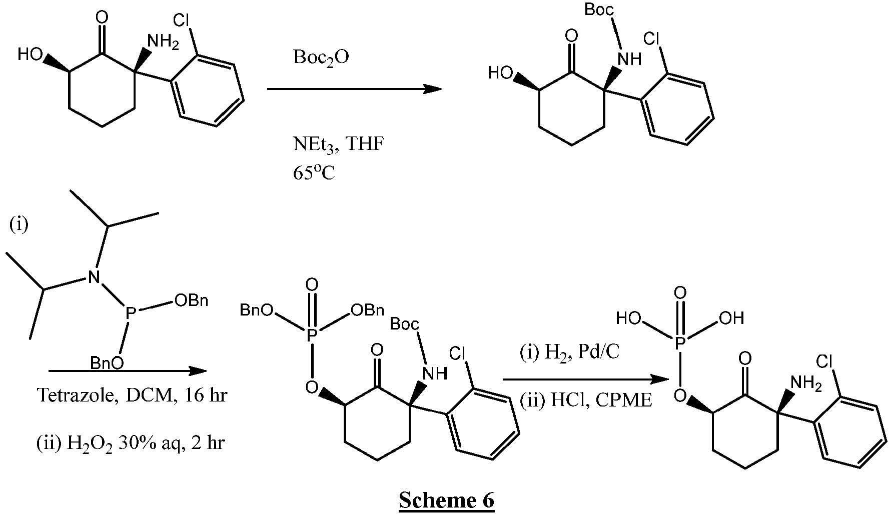

The free acid form of CI was isolated as a crystalline salt, and the potassium salt was obtained, also as a crystalline salt, following neutralisation for 1 hour with aqueous KHCO₃, followed by lyophilisation.

Structure of CI confirmed by ¹H and ³¹P NMR: **¹H-NMR** (301 MHz, METHANOL-D4) δ 7.69-7.72 (m, 1H), 7.29-7.43 (m, 3H), 4.69-4.77 (m, 1H), 2.91-2.97 (m, 1H), 2.52-2.61 (m, 1H), 1.57-1.82 (m, 4H), (NH₂ appears to be under H₂O signal ~4.85 ppm);**³¹P-NMR** (162 MHz, METHANOL-D4) δ 1.87, 0.50.

### Example 3: Solid oral dosage formulations of 2R,6R-hydroxynorketamine

### Formulation (a)

| **2R,6R-HNK Capsule** | | |
|---|---|---|
| **Ingredient/Component** | **Unit Quantity** | **Reference** |
| 2R,6R-hydroxynorketamine (+)-tartrate | 65 mg (40 mg as free base) | In-house |
| Dicalcium phosphate | 648 mg | Ph.Eur |
| Sodium lauryl sulphate | 15 mg | Ph.Eur |
| Colloidal silica | 7 mg | Ph.Eur |
| Coni-Snap^{®} Size 00 hard gelatin capsule or Vcap^{®} Size 00 HPMC capsule | One | In-house |
| Total | 735 mg | |

### Formulation (b)

| **2R,6R-HNK Capsule** | | |
|---|---|---|
| **Ingredient/Component** | **Unit Quantity** | **Reference** |
| 2R,6R-hydroxynorketamine (+)-tartrate | 65 mg (40 mg as free base) | In-house |
| Dicalcium phosphate | 577 mg | Ph.Eur |
| Starch | 58 mg | Ph.Eur |
| Talc | 7 mg | Ph.Eur |
| Hypromellose | 28 mg | Ph.Eur |
| Coni-Snap^{®} Size 00 hard gelatin capsule or Vcap^{®} Size 00 HPMC capsule | One | In-house |
| Total | 735 mg | |

### Formulation (c)

| **2R,6R-HNK Capsule** | | |
|---|---|---|
| **Ingredient/Component** | **Unit Quantity** | **Reference** |
| 2R,6R-hydroxynorketamine as free base | 65 mg (40 mg as free base) | In-house |
| Dicalcium phosphate | 407 mg | Ph.Eur |
| Microcrystalline cellulose | 190 mg | Ph.Eur |
| Crospovidone | 59 mg | Ph.Eur |
| Colloidal silica | 7 mg | Ph.Eur |
| Sodium lauryl sulphate | 7 mg | Ph.Eur |
| Coni-Snap^{®} Size 00 hard gelatin capsule or Vcap^{®} Size 00 HPMC capsule | One | In-house |
| Total | 735 mg | |

### Formulation (d)

| **2R,6R-HNK Capsule** | | |
|---|---|---|
| **Ingredient/Component** | **Unit Quantity** | **Reference** |
| 2R,6R-hydroxynorketamine as free base | 65 mg (40 mg as free base) | In-house |
| Starch | 670 mg | Ph.Eur |
| Coni-Snap^{®} Size 00 hard gelatin capsule or Vcap^{®} Size 00 HPMC capsule | One | In-house |
| Total | 735 mg | |

### Example 4: Analysis of composition of solid oral dosage forms

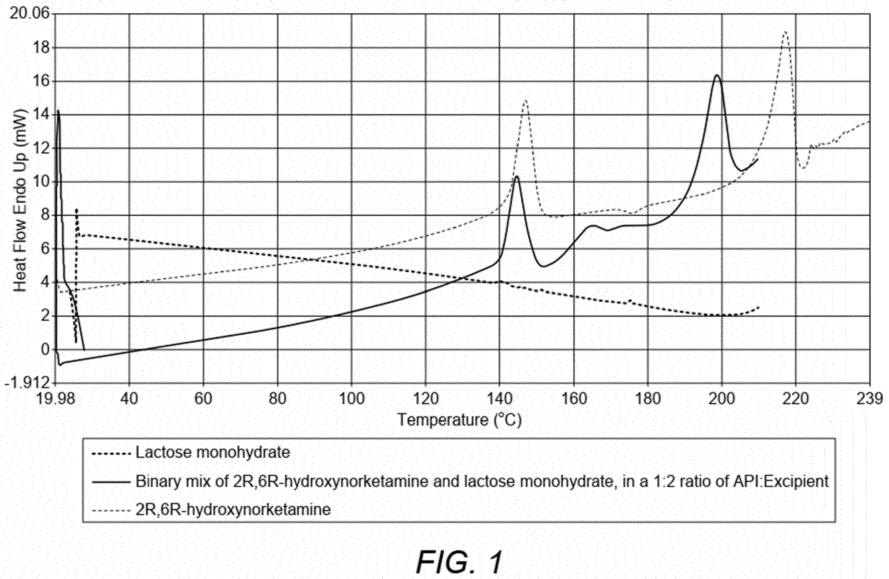

Differential Scanning Calorimetry (DSC) analysis was performed to determine the preferred diluent blend for formulating solid oral dosage forms of the present invention. The results indicate that, despite its widespread use in preparation of solid oral dosage forms, lactose monohydrate is incompatible with 2R,6R-hydroxynorketamine (see Figure 1). These data demonstrated compatibility of 2R,6R-hydroxynorketamine with dicalcium phosphate and microcrystalline cellulose.

Analysis also demonstrated compatibility of 2R,6R-hydroxynorketamine with gelatin and hydroxypropyl methylcellulose capsule shells.

### Example 5: Lack of drug-drug interactions (DDIs) between ketamine metabolites and serotonin modulators such as SSRIs

### Example 5a.

Potential interactions between ketamine metabolites of the present invention with the serotonergic system were investigated to determine whether co-administration with serotonin modulators, especially selective serotonin-reuptake inhibitors (SSRIs) affects the risk of serotonin syndrome.

Serotonin syndrome describes a specific set of symptoms resulting from an excess of serotonin within the central and peripheral nervous systems. Symptoms can vary largely, and range in severity, including possible increases in temperature, agitation and tremor, through to arrhythmias and seizure.

SSRIs work by inhibiting the reuptake of neuronal serotonin, thus increasing the synaptic concentration of the neurotransmitter and therefore activation of SHT receptors. No one 5HT receptor is thought to be responsible for the development of serotonin syndrome, although much focus has highlighted the importance of 5HT_{2A} receptors in the development of this serotonin related toxicity. The risk of serotonin syndrome is increased if SSRIs are taken in combination with another drug that increases extracellular serotonin, or that additively potentiates the SHT receptors involved in its development.

2R,6R-hydroxynorketamine was investigated for binding to monoamine oxidases A and B, monoamine transporters, as well as a variety of 5HT receptors. It was found that 2R,6R-hydroxynorketamine does not interact with transporters or enzymes that directly affect synaptic monoamine concentration. In addition, studies revealed that 2R,6R-hydroxynorketamine does not bind 5HT_{1A}, 5HT_{1B}, 5HT_{2A}, 5HT_{2B} or 5HT_{2C} receptor subtypes, indicating no risk of additive, or indeed competitive, effects if given in combination with SSRIs.

**Table 9**

| **Assay** | **% Inhibition of Control Specific Binding** | ***% of Control Specific Binding*** |
|---|---|---|
| 5-HT1A | 5 | *97.9* |
| 5-HT1B | -2 | *102.7* |
| 5-HT2A | 0 | *103.8* |
| 5-HT2B | 5 | *100.9* |
| 5-HT2C | 10 | *91.1* |
| norepinephrine transporter | -16 | *117.6* |
| dopamine transporter | 11 | *87.2* |
| 5-HT transporter | 2 | *102.0* |
| 5-HT1, Non-Selective | -9 | *105.4* |
| MAO-A | -5 | *97.7* |
| MAO-B | -1 | *100.4* |

An absence of interaction between 2R,6R-hydroxynorketamine and 5HT₁ receptors is also of note. Rare postmarketing reports have described patients with weakness, hyperreflexia, and incoordination following the use of an SSRI and the 5HT₁ agonist, sumatriptan. The possibility of such interactions should also be considered if other 5HT₁ agonists are to be used in combination with SSRIs. As 2R,6R-hydroxynorketamine does not bind 5HT₁ receptors, this potential drug-drug interaction (DDI) is avoided.

### Example 5b.

The p-gycloprotein-1 (p-gp) transporter mediates the efflux of drugs from cells. It is widely expressed throughout the body, including the luminal membrane of the small intestine, apical membranes of hepatocytes and kidney proximal tube epithelia, as well as the blood brain barrier (BBB). It is through its role at the BBB that p-gp plays a key role in limiting drug entry to the central nervous system. Many of the most commonly used antidepressants have been shown to be substrates of p-gp; including: amitriptyline, citalopram, desipramine, doxepine, fluoxetine, fluvoxamine, imipramine, nortriptyline, paroxetine, trimipramine, venlafaxine.

2R,6R-hydroxynorketamine was tested using a bidirectional transcellular transport assay (Caco-2). No active efflux of 2R,6R-hydroxynorketamine was observed (efflux ratio <2, Table 10) indicating that 2R,6R-hydroxynorketamine is not a substrate of either p-gp or Breast Cancer Resistance Protein (BCRP) efflux transporters. In contrast to the effect on p-gp substrate, talinolol, the transporter inhibitor, elacridar, did not affect the efflux ratio of 2R,6R-hydroxynorketamine (2R,6R-hydroxynorketamine efflux ratio = 1.11, + elacridar = 1.15; talinolol efflux ratio = 37, + elacridar = 0.882).

This finding eliminates the possibility that combination therapy of 2R,6R-hydroxynorketamine and an SSRI described above, would induce competition for the p-gp transporter. Such a finding is relevant for the prevention of DDIs involving increased concentrations of either compound within the brain.

Many SSRIs also act as inhibitors of p-gp transporters, with sertraline and paroxetine displaying an IC₅₀ similar to that of established p-gp inhibitor quinidine. As 2R,6R-hydroxynorketamine is not a substrate of p-gp, combination therapy with these SSRIs will not induce DDIs based on a reduction in 2R,6R-hydroxynorketamine efflux from the brain.

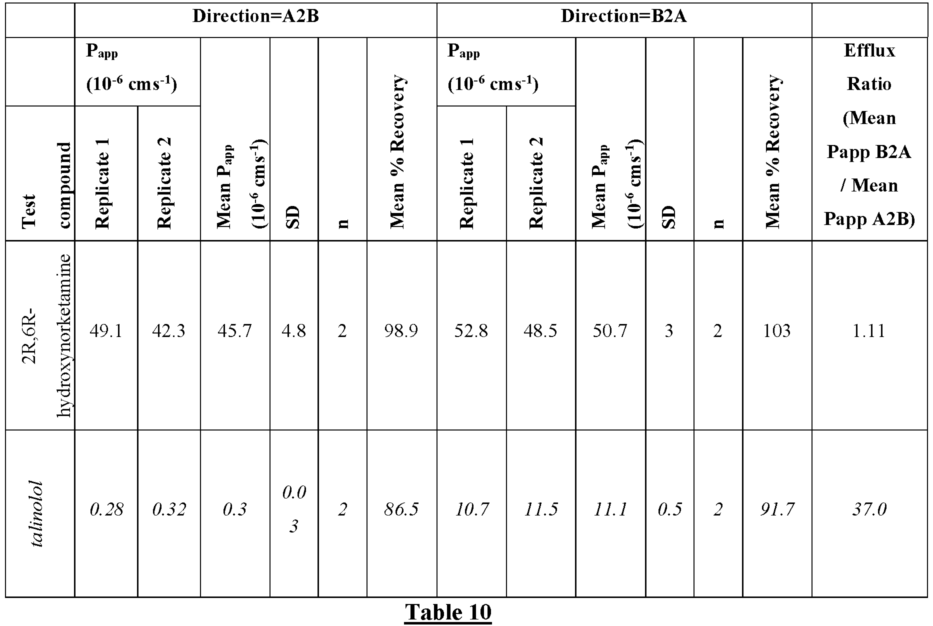

### Example 5c.

While the risk of DDIs due to displacement from plasma binding proteins (PPBs) is relatively low, it should be considered for drugs which display a high proportion PPB (fraction unbound (fu)<1%).

Many commonly used SSRIs are highly protein bound (fluoxetine: 94%; paroxetine: 95%; sertraline: 98%), with advice that co-administration with another drug that also displays high protein binding may result in adverse effects due to an increase in plasma levels of either unbound drug.

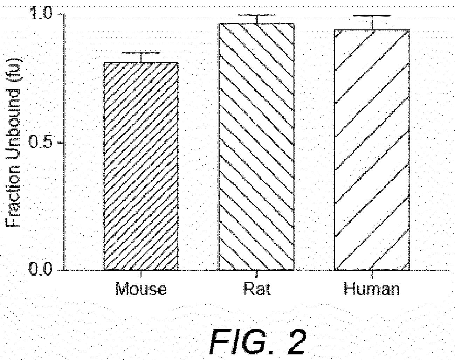

The fu was investigated for 2R,6R-hydroxynorketamine using a 100% plasma from 4 test species. 2R,6R-hydroxynorketamine was found to display low PPB in all species tested (mouse, rat, dog and human; Figure. 2).

As a small molecule with low levels of PPB, 2R,6R-hydroxynorketamine does not risk displacement of SSRIs.

### Example 5d.

Cytochrome P450s (CYPs) are a family of enzymes that play a primary role in the metabolism of a wide variety of drugs. 2R,6R-hydroxynorketamine was investigated for its potential to inhibit the action of 7 key CYP enzymes using an inhibition assay measuring reduction in the formation of metabolites compared to control. These data were then used to calculate an IC₅₀ value.

2R,6R-hydroxynorketamine did not inhibit cytochrome P450 isoforms: CYP2B6, CYP2C8, CYP2C9, CYP2C19 and CYP2D6 (Table 11). 2R,6R-hydroxynorketamine displayed inhibition of CYP1A2, with an IC₅₀ of ~100µM (Table 11). 2R,6R-hydroxynorketamine was found to inhibit the action of CYP3A4 on both substrate groups, midazolam and testosterone, with an IC₅₀ of 69µM and 81µM respectively (Table 11).

**Table 11**

| **Cytochrome P450** | **% Inhibition** | | | | | | |
|---|---|---|---|---|---|---|---|
| | **0 µM** | **0.4 µM** | **1 µM** | **4 µM** | **10 µM** | **40 µM** | **100 µM** |
| CYP2D6 | 0 | 3.16 | 6.65 | 11.5 | 9.41 | 11.2 | 5.73 |
| CYF2C19 | 0 | -2.01 | -1.66 | 1.85 | 6.69 | 8.25 | 16.1 |
| CYP2C8 | 0 | 5.63 | 4.91 | 12.5 | 8.33 | 8.35 | 2.08 |
| CYP2C9 | 0 | 4.59 | 5.92 | 5.80 | 11.9 | -0.113 | -1.33 |
| CYP2B6 | 0 | 6.03 | 10.1 | 10.6 | 14.4 | 14.8 | 16.7 |
| CYP1A2 | 0 | 7.55 | 8.80 | 11.7 | 19.3 | 24.8 | 48.6 |
| CYP3A4, Substrate = midazolam | 0 | 11.5 | 8.44 | 15.7 | 25.7 | 43.7 | 57.5 |
| CYP3A4, Substrate = testosterone | 0 | 1.62 | -0.509 | 7.04 | 12.4 | 35.2 | 53.4 |

CYP2D6 is the major enzyme involved in the metabolism of the majority of commonly used antidepressants, including fluoxetine, paroxetine and venlafaxine. 2R,6R-hydroxynorketamine does not cause inhibition of this CYP enzyme, evading potential DDIs due to decreased metabolism of such SSRIs.

The free (unbound) concentration of 2R,6R-hydroxynorketamine producing pharmacodynamics activity in the brain is estimated to be approximately 10µM. This concentration has been demonstrated to be sufficient in causing an increase in field excitatory postsynaptic potentials (fEPSPs) in *in vitro* electrophysiological experiments, a finding is dependent on the potentiation of α-amino-3-hydroxy-5-methyl-4-isoxazolepropionic acid receptor (AMPAR)-mediated currents. A free concentration 10µM 2R,6R-hydroxynorketamine is sufficiently remote from the IC₅₀ values for CYP1A2 and CYP3A4 to avoid DDI risk.

### Example 5e.

Following phase I metabolism by CYP enzymes, many drugs undergo phase II metabolism, including glucuronidation, a process completed by the uridine glucuronyl transferase (UGT) family.

The SSRI sertraline is glucuronidated to sertraline N-carbamoyl glucuronide by a variety of UGT enzymes, with the greatest activity observed with UGT2B7. 2R,6R-hydroxynorketamine was therefore investigated for its potential to inhibit this isozyme, along with that of the major UGT isozyme, UGT1A1, using a UGT inhibition assay.

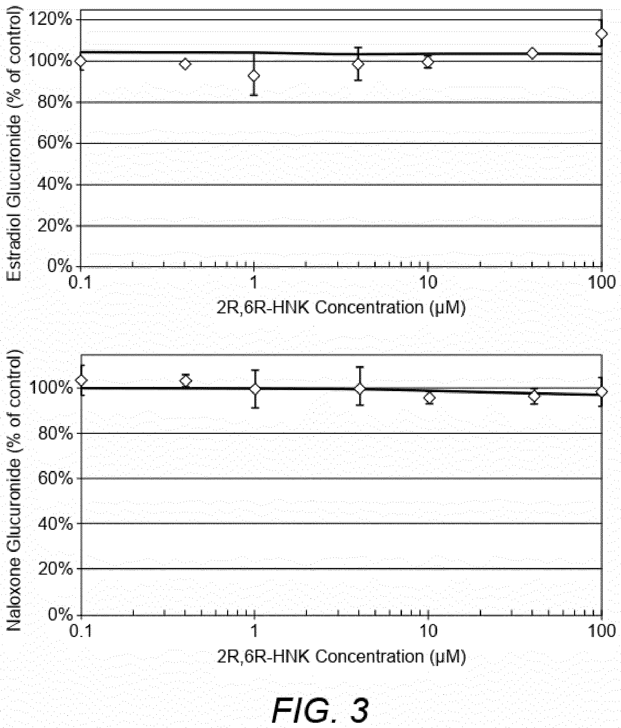

2R,6R-hydroxynorketamine does not inhibit UGT2B7 or UGT1A1 (Table 12, Figure 3), supporting the possibility of its use in combination with sertraline.

**Table 12**

| **UGT Inhibition: Summary of IC50 Values (µM)** | | | |
|---|---|---|---|
| **Inhibitor** | **Test Concentration** | **UGT1A1** | **UGT2B7** |
| **2R-6R-Hydroxynorketamine Hydrochloride** | 0.1µM - 100µM | > 100 | > 100 |
| *Atazanavir* | *0.006µM - 6µM* | *0.153* | |
| *Diclofenac* | *2.7µM - 2000µM* | | *16.4* |

### Example 6: Demonstration that 2R,6R-hydroxynorketamine is orally bioavailable

The solubility and permeability of 2R,6R-hydroxynorketamine was investigated using turbidimetric aqueous solubility and Caco-2 permeability assays respectively. 2R,6R-hydroxynorketamine was found to display high solubility (estimated precipitation range lower bound: 100µM; upper bound: >100µM) and permeability (Table 13).

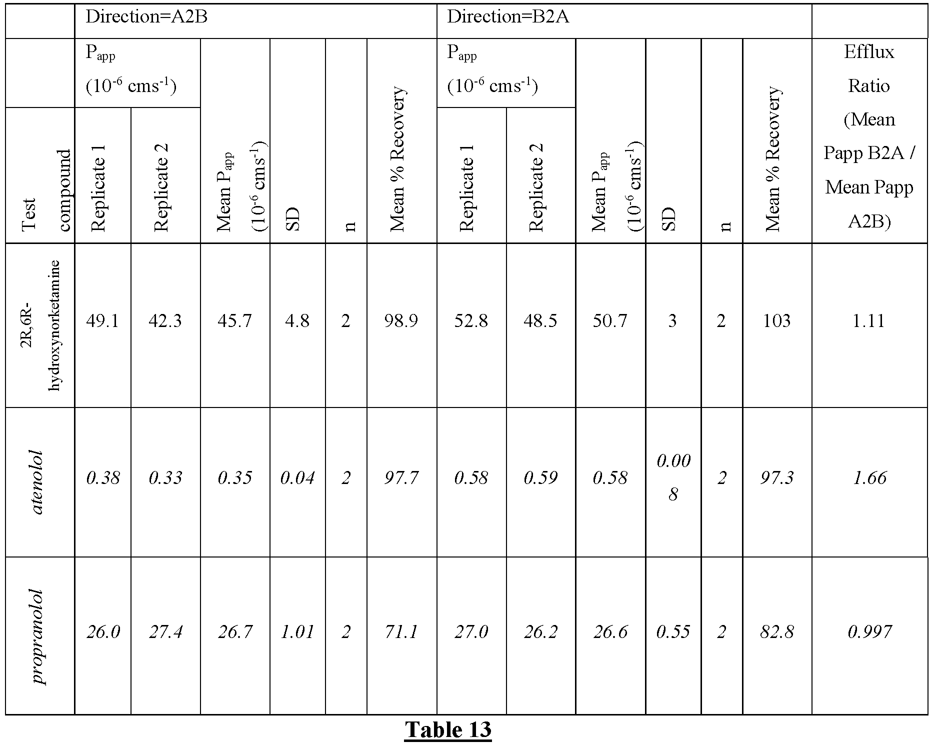

Propranolol has a known human absorption of 90%. Mean apparent permeability coefficient (Papp) values for 2R,6R-hydroxynorketamine are greater than those seen for propranolol controls, suggestive of high human absorption.

Both the solubility and permeability of a compound play a major role in its suitability for oral administration. The data reported presently support 2R,6R-hydroxynorketamine as a suitable candidate for oral administration.

Following absorption, orally administered drugs undergo presystemic metabolism. This first-pass effect contributes one of largest factors affecting the oral bioavailability of a drug.

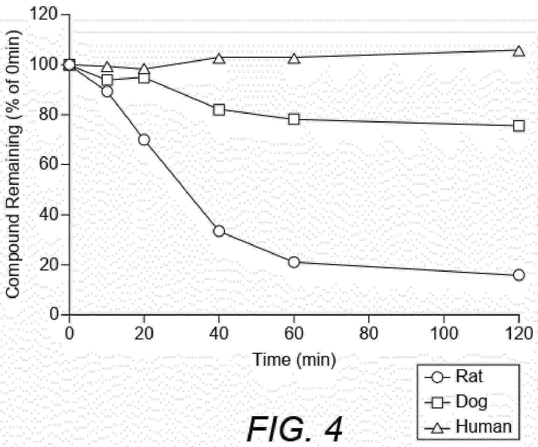

2R,6R-hydroxynorketamine was investigated using an *in vitro* hepatocyte stability assay, during which 2R,6R-hydroxynorketamine was incubated with cryopreserved hepatocytes from 3 test species. 2R,6R-hydroxynorketamine displayed a negative intrinsic clearance value (Clᵢₙₜ) in a human hepatocyte stability assay, indicating it does not undergo hepatic clearance in humans (Table 14, Figure 4). These data, along with metabolite profiling revealed that 2R,6R-hydroxynorketamine does not undergo phase I metabolism by CYP enzymes, but is glucuronidated and excreted via the renal system.

**Table 14**

| Species | CLᵢₙₜ (µL/min/10⁶ cells) | SE CLᵢₙₜ | t_{1/2} (min) | n |
|---|---|---|---|---|
| Rat | 55.2 | 4.24 | 25.1 | 5 |
| Dog | 4.72 | 1.15 | 294 | 6 |
| Human | -1.07 | 0.293 | -1300 | 6 |

The absence of phase I metabolism is supportive of 2R,6R-hydroxynorketamine being suitable for oral administration.

## Claims

1. A solid oral dosage form for use as a first-line therapy in treating a depressive disorder in a patient, said dosage form comprising 2R,6R-hydroxynorketamine or a pharmaceutically acceptable salt thereof, or a prodrug of 2R,6R-hydroxynorketamine having a structure of Formula Ib, or a pharmaceutically acceptable salt thereof, 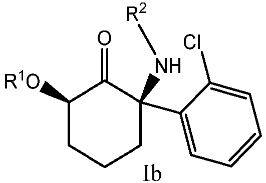 wherein:
(i) R¹ is selected from PO₃H₂, SO₃H, CO₂H, and R⁵, wherein R⁵ is selected from 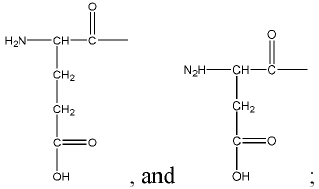 R² is H; or
(ii) R¹ is H;
R² is selected from CH₂OPO₃H₂, CH₂OSO₃H and R⁴, wherein R⁴ is selected from the groups listed in Table 1; 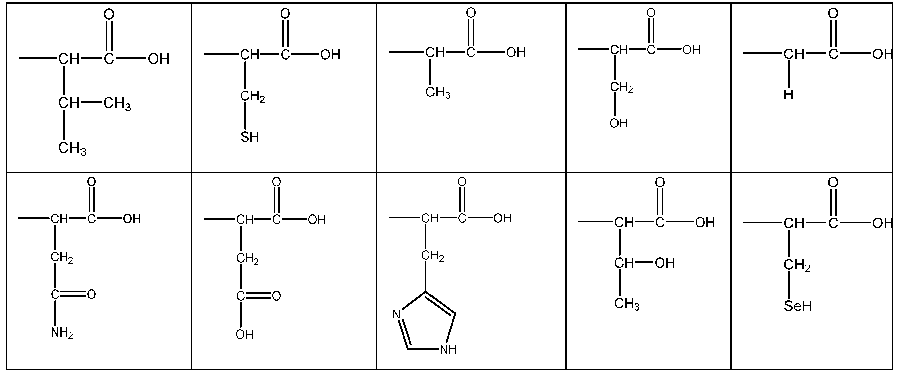 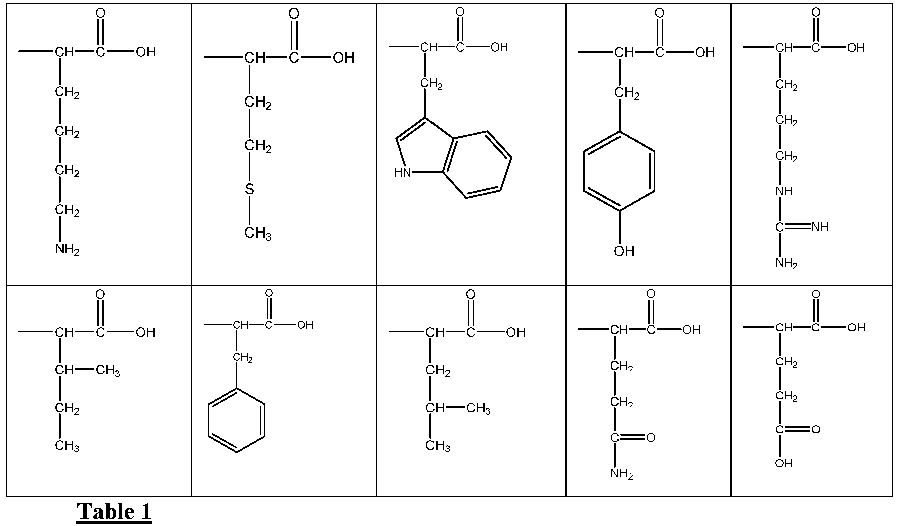
wherein the solid oral dosage form is for administration in combination with a serotonin modulator.

2. The solid oral dosage form for use of claim 1 comprising between 10 mg and 100mg of 2R,6R-hydroxynorketamine, or the equivalent thereof.

3. The solid oral dosage form for use of claim 1 or 2 comprising a crystalline form of 2R,6R-hydroxynorketamine or a pharmaceutically acceptable salt thereof, or a crystalline form of a prodrug of 2R,6R-hydroxynorketamine or a pharmaceutically acceptable salt thereof.

4. The solid oral dosage form for use of any one of claims 1 to 3 wherein the dosage form is a capsule or a tablet.

5. The solid oral dosage form for use of any one of claims 1 to 4 comprising a blend of one or more diluent.

6. The solid oral dosage form for use of claim 5 wherein the dosage form is a tablet and wherein the blend of one or more diluent comprises microcrystalline cellulose.

7. The solid oral dosage form for use of claim 5 or claim 6 wherein the blend of one or more diluent does not contain lactose monohydrate.

8. The solid oral dosage form for use of any one of claims 1 to 5 or 7 wherein the dosage form is a capsule and the capsule shell comprises a constituent selected from gelatin and hydroxypropyl methylcellulose.

9. The solid oral dosage form for use of any one of claims 1 to 8 wherein the serotonin modulator is a selective serotonin reuptake inhibitor (SSRI).

10. The solid oral dosage form for use of any one of claims 1 to 9 wherein said solid oral dosage form and said serotonin modulator are administered simultaneously, sequentially or separately.

11. The solid oral dosage form for use of any one of claims 1 to 10 wherein said serotonin modulator is selected from Citalopram, Escitalopram, Paroxetine, Fluoxetine, Fluvoxamine, Sertraline, Desvenlafaxine, Duloxetine, Levomilnacipran, Milnacipran, Tofenacin, Venlafaxine, Vilazodone, Vortioxetine, Etoperidone, Nefazodone, and Trazodone, or a pharmaceutically acceptable salt thereof, preferably selected from Citalopram, Escitalopram, Paroxetine, Sertraline, Duloxetine, and Venlafaxine, or a pharmaceutically acceptable salt thereof.

12. The solid oral dosage form for use of any one of claims 1 to 11 wherein the patient has experienced one or more manic episode or hypomanic episode, is suffering a manic episode or hypomanic episode, or is at risk of suffering one or more manic episode or hypomanic episode; and/or wherein the patient is suffering from bipolar depression type 1, bipolar depression type 2, bipolar depression, or obsessive-compulsive disorder comorbid with depression.

13. The solid oral dosage form for use of any one of claims 1 to 12 wherein said pharmaceutically acceptable salt is a tartrate salt.

14. The solid oral dosage form for use of claim 13 comprising 2R,6R-hydroxynorketamine L-(+)-tartrate.

## Patentansprüche

1. Feste orale Darreichungsform zur Verwendung als First-Line-Therapie beim Behandeln einer depressiven Störung bei einem Patienten, wobei die Darreichungsform 2R,6R-Hydroxynorketamin oder ein pharmazeutisch annehmbares Salz davon oder ein Prodrug von 2R,6R-Hydroxynorketamin mit einer Struktur von Formel Ib oder ein pharmazeutisch annehmbares Salz davon umfasst, 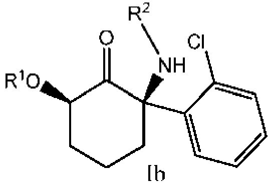 wobei:
(i) R¹ ausgewählt ist aus PO₃H₂, SO₃H, CO₂H und R⁵, wobei R⁵ ausgewählt ist
aus 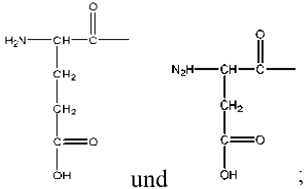 R² H ist; oder
(ii) R¹ H ist;
R² ausgewählt ist aus CH₂OPO₃H₂, CH₂OSO₃H und R⁴, wobei R⁴ ausgewählt ist aus den Gruppen aufgelistet in Tabelle 1; 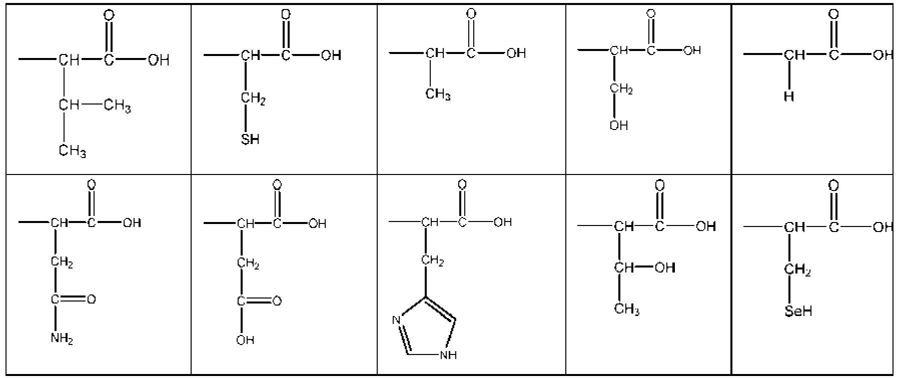 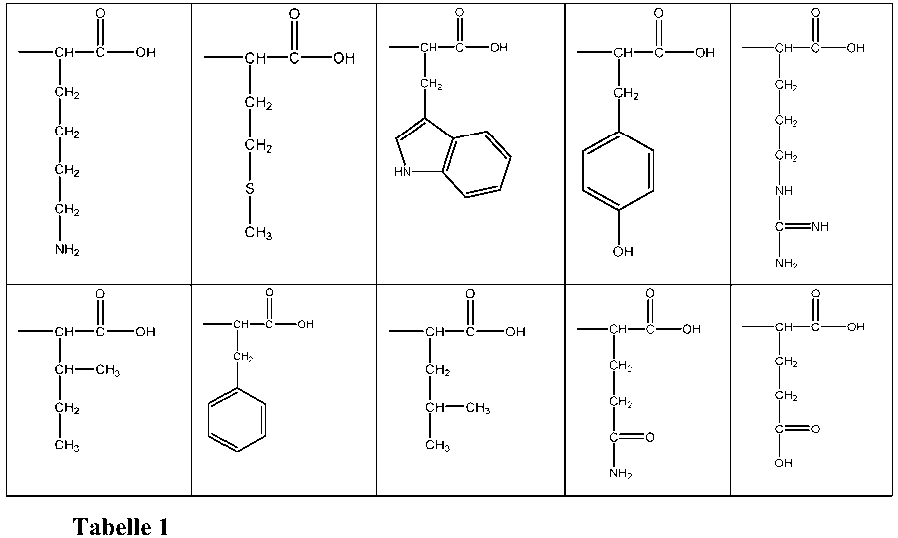
wobei die feste orale Darreichungsform zur Verabreichung in Kombination mit einem Serotoninmodulator ist.

2. Feste orale Darreichungsform zur Verwendung nach Anspruch 1, umfassend zwischen 10 mg und 100 mg von 2R,6R-Hydroxynorketamin oder dem Äquivalent davon.

3. Feste orale Darreichungsform zur Verwendung nach Anspruch 1 oder 2, umfassend eine kristalline Form von 2R,6R-Hydroxynorketamin oder einem pharmazeutisch annehmbaren Salz davon oder eine kristalline Form eines Prodrugs von 2R,6R-Hydroxynorketamin oder einem pharmazeutisch annehmbaren Salz davon.

4. Feste orale Darreichungsform zur Verwendung nach einem der Ansprüche 1 bis 3, wobei die Darreichungsform eine Kapsel oder eine Tablette ist.

5. Feste orale Darreichungsform zur Verwendung nach einem der Ansprüche 1 bis 4, umfassend eine Mischung aus einem oder mehreren Verdünnungsmitteln.

6. Feste orale Darreichungsform zur Verwendung nach Anspruch 5, wobei die Darreichungsform eine Tablette ist und wobei die Mischung aus einem oder mehreren Verdünnungsmitteln mikrokristalline Cellulose umfasst.

7. Feste orale Darreichungsform zur Verwendung nach Anspruch 5 oder Anspruch 6, wobei die Mischung aus einem oder mehreren Verdünnungsmitteln kein Lactosemonohydrat enthält.

8. Feste orale Darreichungsform zur Verwendung nach einem der Ansprüche 1 bis 5 oder 7, wobei die Darreichungsform eine Kapsel ist und die Kapselhülle einen Bestandteil ausgewählt aus Gelatine und Hydroxypropylmethylcellulose umfasst.

9. Feste orale Darreichungsform zur Verwendung nach einem der Ansprüche 1 bis 8, wobei der Serotoninmodulator ein selektiver Serotoninwiederaufnahmehemmer (SSRI) ist.

10. Feste orale Darreichungsform zur Verwendung nach einem der Ansprüche 1 bis 9, wobei die feste orale Darreichungsform und der Serotoninmodulator gleichzeitig, nacheinander oder getrennt verabreicht werden.

11. Feste orale Darreichungsform zur Verwendung nach einem der Ansprüche 1 bis 10, wobei der Serotoninmodulator ausgewählt ist aus Citalopram, Escitalopram, Paroxetin, Fluoxetin, Fluvoxamin, Sertralin, Desvenlafaxin, Duloxetin, Levomilnacipran, Milnacipran, Tofenacin, Venlafaxin, Vilazodon, Vortioxetin, Etoperidon, Nefazodon und Trazodon oder einem pharmazeutisch annehmbaren Salz davon, bevorzugt ausgewählt aus Citalopram, Escitalopram, Paroxetin, Sertralin, Duloxetin und Venlafaxin oder einem pharmazeutisch annehmbaren Salz davon.

12. Feste orale Darreichungsform zur Verwendung nach einem der Ansprüche 1 bis 11, wobei der Patient eine oder mehrere manische Episoden oder hypomanische Episoden erlebt hat, an einer manischen Episode oder hypomanischen Episode leidet oder bei dem das Risiko besteht, an einer oder mehreren manischen Episoden oder hypomanischen Episoden zu leiden; und/oder wobei der Patient an bipolarer Depression Typ 1, bipolarer Depression Typ 2, bipolarer Depression oder Zwangsstörung einhergehend mit Depression leidet.

13. Feste orale Darreichungsform zur Verwendung nach einem der Ansprüche 1 bis 12, wobei das pharmazeutisch annehmbare Salz ein Tartratsalz ist.

14. Feste orale Darreichungsform zur Verwendung nach Anspruch 13, umfassend 2R,6R-Hydroxynorketamin-L-(+)-tartrat.

## Revendications

1. Forme posologique solide orale pour utilisation comme thérapie de première intention dans le traitement d'un trouble dépressif chez un patient, ladite forme posologique comprenant de la 2R,6R-hydroxynorkétamine ou un sel pharmaceutiquement acceptable de celle-ci, ou un promédicament de la 2R,6R-hydroxynorkétamine présentant une structure de Formule Ib, ou un sel pharmaceutiquement acceptable de celui-ci, 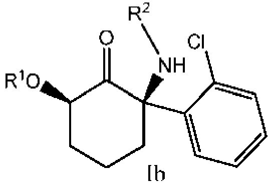 dans laquelle :
(i) R¹ est choisi parmi PO₃H₂, SO₃H, CO₂H et R⁵, où R⁵ est choisi parmi 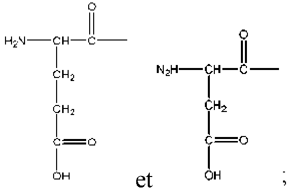 R² est H ; ou
(ii) R¹ est H ;
R² est choisi parmi CH₂OPO₃H₂, CH₂OSO₃H et R⁴, où R⁴ est choisi parmi les groupes énumérés dans le Tableau 1 ; 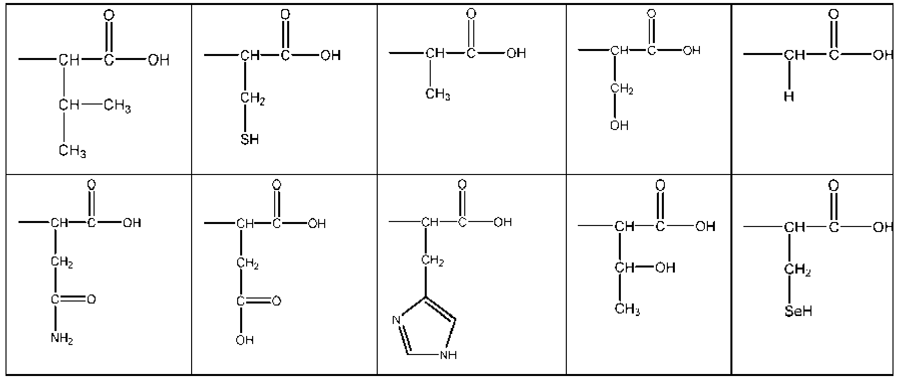 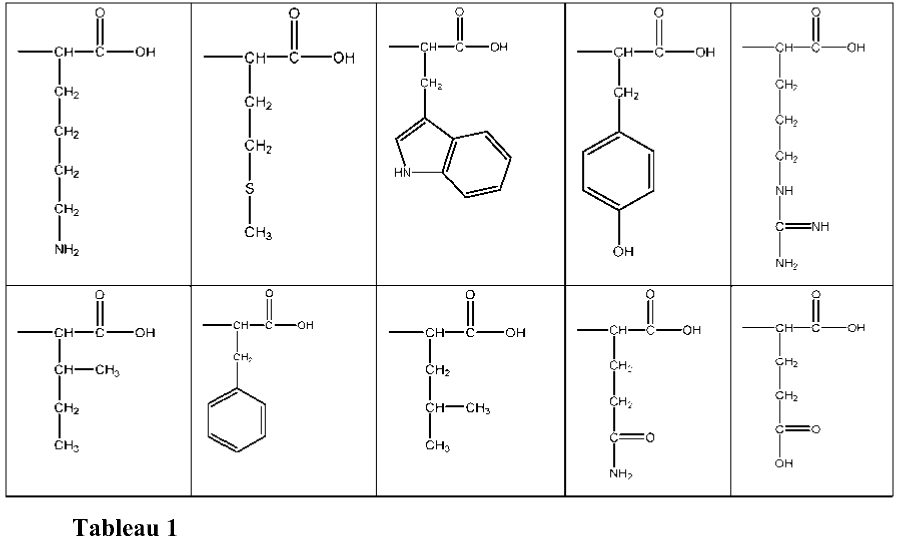
dans laquelle la forme posologique solide orale est destinée à être administrée en combinaison avec un modulateur de sérotonine.

2. Forme posologique solide orale pour utilisation selon la revendication 1, comprenant entre 10 mg et 100 mg de 2R,6R-hydroxynorkétamine, ou l'équivalent de celle-ci.

3. Forme posologique solide orale pour utilisation selon l'une des revendications 1 ou 2, comprenant une forme cristalline de 2R,6R-hydroxynorkétamine ou d'un sel pharmaceutiquement acceptable de celle-ci, ou une forme cristalline d'un promédicament de 2R,6R-hydroxynorkétamine ou d'un sel pharmaceutiquement acceptable de celui-ci.

4. Forme posologique solide orale pour utilisation selon l'une quelconque des revendications 1 à 3, dans laquelle la forme posologique est une capsule ou un comprimé.

5. Forme posologique solide orale pour utilisation selon l'une quelconque des revendications 1 à 4, comprenant un mélange d'un ou plusieurs diluants.

6. Forme posologique solide orale pour utilisation selon la revendication 5, dans laquelle la forme posologique est un comprimé et dans laquelle le mélange d'un ou plusieurs diluants comprend de la cellulose microcristalline.

7. Forme posologique solide orale pour utilisation selon la revendication 5 ou la revendication 6, dans laquelle le mélange d'un ou plusieurs diluants ne contient pas de lactose monohydraté.

8. Forme posologique solide orale pour utilisation selon l'une quelconque des revendications 1 à 5 ou 7, dans laquelle la forme posologique est une capsule et l'enveloppe de capsule comprend un constituant choisi parmi la gélatine et l'hydroxypropylméthylcellulose.

9. Forme posologique solide orale pour utilisation selon l'une quelconque des revendications 1 à 8, dans laquelle le modulateur de sérotonine est un inhibiteur sélectif de la recapture de la sérotonine (ISRS).

10. Forme posologique solide orale pour une utilisation selon l'une quelconque des revendications 1 à 9, dans laquelle ladite forme posologique solide orale et ledit modulateur de sérotonine sont administrés simultanément, séquentiellement ou séparément.

11. Forme posologique solide orale pour utilisation selon l'une quelconque des revendications 1 à 10, dans laquelle ledit modulateur de sérotonine est choisi parmi le Citalopram, l'Escitalopram, la Paroxétine, la Fluoxétine, la Fluvoxamine, la Sertraline, la Desvenlafaxine, la Duloxétine, le Lévomilnacipran, le Milnacipran, la Tofénacine, la Venlafaxine, la Vilazodone, la Vortioxétine, l'Étopéridone, la Néfazodone et la Trazodone, ou un sel pharmaceutiquement acceptable de ceux-ci, de préférence choisi parmi le Citalopram, l'Escitalopram, la Paroxétine, la Sertraline, la Duloxétine et la Venlafaxine, ou un sel pharmaceutiquement acceptable de ceux-ci.

12. Forme posologique solide orale pour utilisation selon l'une quelconque des revendications 1 à 11, dans laquelle le patient a connu un ou plusieurs épisodes maniaques ou épisodes hypomaniaques, souffre d'un épisode maniaque ou d'un épisode hypomaniaque, ou risque de souffrir d'un ou plusieurs épisodes maniaques ou épisodes hypomaniaques ; et/ou dans laquelle le patient souffre d'une dépression bipolaire de type 1, d'une dépression bipolaire de type 2, d'une dépression bipolaire ou d'un trouble obsessionnel-compulsif comorbide avec une dépression.

13. Forme posologique solide orale pour utilisation selon l'une quelconque des revendications 1 à 12, dans laquelle ledit sel pharmaceutiquement acceptable est un sel de tartrate.

14. Forme posologique solide orale pour utilisation selon la revendication 13, comprenant du L-(+)-tartrate de 2R,6R-hydroxynorkétamine.
